Europäisches Patentamt

European Patent Office

Office européen des brevets

(11) Publication number: **0 231 624 B1**

(12) **EUROPEAN PATENT SPECIFICATION**

(45) Date of publication of patent specification: **27.01.93**

(21) Application number: **86309704.4**

(22) Date of filing: **12.12.86**

Consolidated with 87900529.6/0289508
(European application No./publication No.) by
decision dated 28.12.89.

The file contains technical information submitted
after the application was filed and not included in
this specification

(51) Int. Cl.⁵: **C12N 15/58**, C12N 9/64,
C12N 1/20, C12N 1/16,
C12N 5/00

(54) Tissue plasminogen activator (TPA) analogs.

(30) Priority: **20.12.85 US 811607**
**19.09.86 US 909482**

(43) Date of publication of application:
**12.08.87 Bulletin 87/33**

(45) Publication of the grant of the patent:
**27.01.93 Bulletin 93/04**

(84) Designated Contracting States:
**AT BE CH DE ES FR GB GR IT LI LU NL SE**

(56) References cited:
**EP-A- 213 794**
**EP-A- 0 093 619**
**EP-A- 0 207 589**

**PROCEEDINGS OF THE NATIONAL ACADEMY OF SCIENCES OF THE USA, vol. 81, no. 17, September 1984, pages 5355-5359, Washington, US; T. NY et al.: "The structure of the human tissue-type plasminogen activator gene: Correlation of intron and exon structures to functional and structural domains"**

(73) Proprietor: **THE UPJOHN COMPANY**
**301 Henrietta Street**
**Kalamazoo, Michigan 49001(US)**

(72) Inventor: **Marotti, Keith R.**
**c/o The Upjohn Company 301 Henrietta Street**
**Kalamazoo Michigan 49001(US)**
Inventor: **Rehberg, Edward F.**
**c/o The Upjohn Company 301 Henrietta Street**
**Kalamazoo Michigan 49001(US)**
Inventor: **Theriault, Nicole Y.**
**c/o The Upjohn Company 301 Henrietta Street**
**Kalamazoo Michigan 49001(US)**

(74) Representative: **Perry, Robert Edward et al**
**GILL JENNINGS & EVERY, Broadgate House,**
**7 Eldon Street**
**London EC2M 7LH(GB)**

PROCEEDINGS OF THE NATIONAL ACADEMY OF SCIENCES OF THE USA, vol. 83, no. 13, July 1986, pages 4670-4674, Washington, US; A.J. VAN ZONNEVELD et al.: "Autonomous functions of structural domains on human tissue-type plasminogen activator"

FEBS lett. 189 (1985), 145-149

## Description

This invention discloses human tissue plasminogen activator (TPA) analogs. The analogs are TPA-like molecules that have had the native domain regions either rearranged, deleted, added or a combination thereof. The analogs are the product of the expression of recombinant deoxyribonucleic acid (DNA) also described herein. Additionally, the present invention describes replication and expression plasmids containing the TPA-coding DNA sequences described above and suitable host microorganisms that are capable of expressing the TPA analogs after becoming transformed with an appropriate plasmid.

Background.

TPA has therapeutic value in treating blood clots by helping dissolve the clots as they form. Thrombolysis is the process of fibrin clot dissolution. For that purpose the serine protease plasmin is formed from its inactive zymogen, plasminogen. Activation of plasminogen is achieved through proteolytic cleavage by a class of serine proteases called plasminogen activators. These activators are present in most body fluids and initiate the activation of plasminogen to plasmin. This sets up a cascade whereby a small amount of plasminogen activator can initiate the activation of a large amount of plasminogen. In vivo, the plasminogen activator activates plasminogen to plasmin which then acts to degrade the fibrin clot. The plasminogen activator which is physiologically important to clot lysis is TPA.

TPA is a serine protease with a molecular weight of approximately 66,000 daltons which is produced by the vascular endothelial cells. It is glycosylated and its only known protein substrate is plasminogen. TPA has five domains: the fibronectin finger domain (F), the growth factor domain (G), the kringle 1 domain (K1), the kringle 2 domain (K2) and the active site domain (A). One or more of these domains is responsible for the unique fibrin binding activity of TPA.

In addition to its fibrin affinity, TPA activation of plasminogen is enhanced in the presence of fibrin. Other clot-dissolving drugs such as urokinase or streptokinase do not have any specificity for the fibrin clot. However, the half-life of TPA is estimated at 2-3 minutes, making it inconvenient, if not impractical, to use as a therapeutic agent; see Collen et al, Science 220:1181-1183 (1983).

The enzyme kinetics of TPA have been studied; see Rijken et al, J. Biol. Chem. 257:2920-2925 (1982). The binding and fibrolytic affinity of human TPA for human blood clots over the blod clots of other animals is known; see Korninger et al, Thrombos. Haemostas. 46(2):561-565 (1981). The structural relationship of domains with the fibrinolytic activity and affinity has also been delineated; see Banyai et al, FEBS Lett. 163-(1):37-41 (1983), and Ny et al, Proc. Natl. Acad. Sci. USA 81:5355-5350 (1984).

The expression of TPA by transformed bacteria and yeast is also known. The messenger ribonucleic acid (RNA) of human TPA was first isolated in 1982; see Opdenakker et al, Eur. J. Biol., pp. 269-74 (1982). Bacteria were transformed for expression of TPA by Pennica et al, Nature 301:214-21 (1983). Procedures to transform yeast to express human TPA are described in EP-A-0143081. EP-A-0207589 discloses FK1K2A.

SUMMARY OF THE INVENTION

Novel TPA-like proteins are FK2A, FGK2A or FK2K2A, and have a molecular weight of less than 90,000 daltons. Such proteins have improved half-life and fibrin affinity.

According to a second aspect of the present invention, DNA sequences coding for the novel proteins contain unique restriction sites within the interdomain regions of TPA, which are useful for convenient splitting and rearrangement of the domains. Examples of non-native endonuclease restriction sites that are not present in the nucleotide sequences encoding the domain regions are, with respect to the native arrangement: an XbaI site between nucleotide bases 187 and 198, an HpaI or SphI site or combination thereof between nucleotide bases 328 and 342, an EcoRV, ClaI or SmaI site or combination thereof between nucleotide bases 442 and 462, a BamHI or HpaI site or combination thereof between nucleotide bases 709 and 726, and a MstI, SphI or XmaIII site or combination thereof between nucleotide bases 973 and 997.

Preferably, the sequences of nucleotides in DNA, both upstream and downstream from the sequence encoding the TPA-like protein, render the DNA capable of being maintained by cells of a suitable host. More preferably, the DNA is such that the cells can express the TPA-like protein.

Suitable host microorganisms capable of maintaining the expression and replication plasmids (vectors) and for the expression of the TPA-like proteins are also described herein. For example, for expression and non-expression vectors containing DNA sequences coding for TPA-like proteins, suitable hosts include yeast, Escherichia sp., cell cultures and insects such as Bombyx mori or Spodoptera frugiperda. The preferred host cell is a Chinese hamster ovary (CHO) cell.

DESCRIPTION OF THE INVENTION

This invention involves a series of molecular genetic manipulations that can be achieved in a variety of known ways. Two prototype genes having unique endonuclease restriction sites between the domains of TPA have been deposited in accordance with the Budapest Treaty. The host microorganisms are E. coli strains containing plasmids designated pTPA-B1,2,3,4(a) and pTPA-B1,2,3,4 and illustrated in Charts 10 and 11, respectively. The deposit of pTPA-B1,2,3,4(a) was made with the Northern Regional Research Center, Peoria, Illinois, USA on 29th August 1986 and assigned Accession Number NRRL B-18106. The deposit of pTPA-B1,2,3,4 was made with the Northern Regional Research Center, Peoria, Illinois, USA on 28th November 1986 and assigned Accession Number NRRL B-18142.

The deposited plasmids are convenient starting materials. Various methods given below are available to create the TPA analogs from alternative starting materials and is followed by specific examples of preferred methods.

In summary the necessary genetic manipulations can be described as the obtaining of a cDNA of TPA, the synthesizing of blocks of oligonucleotides containing the coding sequence of the various TPA domains with selected restriction sites in the interdomain regions, the cloning and replication of the domains in E. coli and the expression of the desired TPA analog.

A. Tables

Table 1 illustrates the specific sequence and base numbering positions for synthetic TPA DNA compared to the native cDNA. The native domains are all present in their natural order in Table 1. Table 2 compares the amino acid sequence of the native TPA to a TPA analog having all the natural domains in their normal order (see chart 11). In both tables the top sequences present the native sequence of either nucleotides or amino acids with the lower sequences reflecting the modified TPA. Table 3 presents the oligonucleotides used to synthetically build the TPA analogs. Table 4 compares the native TPA protein to the analog described in chart 11 containing the native order of TPA domains by illustrating the amino acids and nucleotides contained within the domain regions. The interdomain regions are those amino acids lying between the domains as delineated in Table 4 (see Definitions). Throughout this document, the numbered references to either nucleotides or amino acids refer to these tables.

B. General Methods

Generally, the nomenclature and general laboratory procedures required in this application can be found in Maniatis, T. et al., Molecular Cloning A Laboratory Manual, Cold Spring Harbor Laboratory, Cold Spring Harbor, New York, 1982. The manual is hereinafter referred to as Maniatis.

All E. coli strains are grown on Luria broth (LB) with glucose, Difco's Antibiotic Medium #2 and M9 medium supplemented with glucose and acid-hydrolyzed casein amino acids. Strains with resistance to antibiotics were maintained at the drug concentrations described in Maniatis. Transformations were performed according to the method described by Morrison, D.A. (1977), J. of Bact., 132:349-351; or by Clark-Curtiss, J.E. and Curtiss, R., 1983, in Methods in Enzymology, 101:347-362, Wu, R., Grossman, L. and Moldave, K., eds., Academic Press, New York.

All enzymes were used according to the manufacturer's instructions. Colony hybridization is carried out as generally described in Grunstein, M. et al., Proc. Nat. Acad. Sci., 72, vol. 72, pp. 3961-5 (1975) but modified to provide the following method: Nitrocellulose filters are made which contain colonies of the cells prepared above. The filters are then laid, colony-side up, onto a bed of 5-8 thickness of Whatman 3MM paper soaked in denaturing solution consisting of 1.5 M NaCl, 0.5 M NaOH. The denaturant is allowed to diffuse upwards into the colonies for 1.5-2 minutes at which time the filters are transferred to a second bed of 3MM papers soaked in neutralizing solution containing 0.5 M Tris•HCl pH 7.4, 2 x SSC (1 x SSC = 0.15 M NaCl; 0.015 M Na Citrate; pH 7.1) and 25 mM EDTA, for 1-3 minutes. Filters are then thoroughly air dried and baked in vacuo for 2-4 hours at 80° C.

Hybridization conditions for oligonucleotide probes are as previously described by Goeddel, D.V. et al., Nature, vol. 290, pp. 20-26 (1981).

After hybridization, the probe containing solution is removed and saved and the filters are washed in 0.1% SDS, 0.2x SSC for a total of 3 hours with 5 changes of 400 ml each. Filters are thoroughly air dried, mounted, and autoradiographed using Kodak X-OMAT AR® film and Dupont Cronex Lightning Plus®

4

intensifying screens for 16 hours at -70° C.

For sequencing of plasmids, minipreps of plasmid DNA are prepared according to the method of Holmes, D. S. et al., Analyt. Biochem., vol. 114, p. 193 (1981). Dideoxy sequencing is carried out according to Sanger F. et al., "Cloning in single stranded bacteriophage as an aid to rapid DNA sequencing", J. Mol. Biol. 143:161-178 (1977). The dideoxy containing reactions are prepared for electrophoresing by heating to 90° for 2 min, and quenching on ice. 2-3 μl per lane is loaded on an 8% sequencing denaturing polyacrylamide gel prepared and ran according to Sanger and Coulson, "The use of thin acrylamide gels for DNA sequencing", FEBS Lett. 87:107-110, (1978). The gels are exposed at room temperature for 2-4 days, and films (Kodak XAR-5®) are developed as per manufacturers' recommendations.

Nucleotide sizes are given in either kilobases (kb) or basepairs (bp). These are estimates derived from agarose gel electrophoresis.

C. TPA cDNA

TPA cDNA was a convenient source of the active site portion of the nucleic acid sequence. Therefore it was unnecessary to chemically synthesize the active site. TPA cDNA is available from a number of sources. Workers have reported preparing portions of the TPA cDNA from messenger RNA isolated from cell cultures producing large amount of TPA specifically Bowes Melanoma cells. Edlund et al., "Isolation of cDNA Sequences Coding for a Part of Human Tissue Plasminogen Activator, vol. 80, pp. 349-352 (Jan. 1983); Gronow et al., "Production of Human Plasminogen Activators by Cell Culture", Trends in Biotechnology, vol. 1, no. 1, pp. 26-29 (1983); and Pennica et al., "Cloning and Expression of Human Tissue-type Plasminogen Activator cDNA in E. coli.", Nature, vol. 301, pp. 214-21 (20 Jan. 1983).

More specifically, Genentech, Inc., filed a European patent application No. 0093619 on 4 May 1983, relying on the priority applications having U.S. Serial Nos. 374,860; 398,003 and 483,052 having the respective filing dates of May 5, 1982; July 14, 1982 and April 7, 1983 which is hereinafter referred to as the Genentech Application. The Genentech Application discloses E. coli K12 strain 294 with ATCC No. 31446, that is a transformant having a recombinant DNA compound coding for TPA. Additionally the E. coli K12 strain W3110 transformed by rDNA is disclosed in the Genentech Application as ATCC No. 27325 from which extracts of a TPA are also prepared for assay of fibrinolytic activity. Thus, the disclosures include a recombinant DNA compound useful herein. Likewise, disclosure of the Genentech Application provides DHFR$^+$ CHO-KI (ATCC CL 61) cells transformed for amplification also expressing TPA. Thus, again the deposit, ATCC CL61 discloses recombinant DNA nucleotides coding for TPA useful herein.

The disclosure herein of Preparation 2 provides one of skill in the art with an isolation process from among the processes known in the art applicable to the Bowes melanoma cell for obtaining the recombinant DNA compound coding for a TPA. The Bowes melanoma cell is commonly available to the public.

D. Synthesizing the TPA Domains.

As noted above, portions of the TPA analog DNA sequences were prepared from cDNA, however, most of the sequence and even the entire sequence could have been created synthetically. Cost and convenience are the controlling parameters in deciding how to create a particular TPA analog's sequence. By selecting restriction sites not found in the TPA domains of the desired analog and by inserting these restriction sites within the interdomain regions of TPA, one avoids digesting a desired part of the TPA DNA sequence and allows for the easy removal and insertion of each individual domain.

By chemically synthesizing a portion of the TPA gene, one can decide on a base by base process which nucleotides are to be used. The following advantages arise from this process: (1) the minimization of secondary structure in the transcription product; (2) the minimization of AT-GC regions of self-complementarity; and, (3) the placement of unique restriction sites at desirable locations along the cDNA sequence.

Oligonucleotides are chemically synthesized according to the solid phase phosphoramidite triester method first described by Beaucage S.L. and Caruthers M.H. Tetrahedron Letts. 22(20):1859-1862 (1981) using an automated synthesizer; as described in Needham-VanDevanter, D.R. et al., Nucleic Acids Res., 12:6159-6168 (1984). Purification of oligonucleotides was by either native acrylamide gel electrophoresis or by anion-exchange HPLC as described in Pearson, J.D. and Regnier, F.E., J. Chrom., 255:137-149 (1983).

Oligodeoxynucleotides are chemically synthesized in lengths of less than 40 nucleotides. Each fragment is designed to be complementary to its corresponding complementary strand and each double stranded segment contains sticky ends to optimize ligation. The following criteria are considered in the division of the DNA for synthesis of the fragments: 1) The length of the oligonucleotide fragments should be less than 40 bases in length for the ease in chemical synthesis and purification. 2) A minimal of 6 bases "protruding" from the complementing "top" and "bottom" oligonucleotide fragments appears to allow optimal alignment and ligation of the segments. 3) Regions of four bases or more which can associate with either the primary or the complement sequence should be avoided.

The sequence of the synthetic oligonucleotides can be verified using the chemical degradation method of Maxam, A.M. and Gilbert, W., Grossman, L. and Moldave, D., eds., Academic Press, New York, Methods in Enzymology, 65:499-560 (1980). Alternatively, the sequence can be confirmed after the assembly of the oligonucleotide fragments into the double-stranded DNA sequence using the method of Maxam and Gilbert, supra, or the chain termination method for sequencing double-stranded templates of Wallace, R.B., et al., Gene, 16:21-26 (1981).

Eighty-seven separate oligonucleotides (Table 3) were synthesized. The specific assembly strategy disclosed herein was to engineer the first 1099 base pairs coding for the 5' portion of the TPA analog having the selected restriction sites indicated in Chart (11). This synthetic oligonucleotide is then joined to the 3' portion of TPA cDNA at the EcoRI site at position 1287 containing the active site.

To assemble the oligonucleotide fragments into the double-stranded DNA, one can mix all of the 87 fragments together for annealing and ligation. For more efficient ligation, it is better to divide the oligonucleotides into four blocks. After ligation and purification of each block, the four blocks can then be annealed and ligated to yield the final product. Purification of the assembled blocks and the final product can be performed by acrylamide gel electrophoresis as described in Maniatis. To carry out the annealing and ligation, the general methods described in Maniatis are also used.

It may be necessary to provide an initiation codon for the synthetic TPA genes. For purposes of the E. coli system, the initiating codon ATG for coding for F-met is required. The initiating codon can be incorporated into the synthetic gene or be provided by the desired expression plasmid. In addition to an initiation codon, it is, for E. coli, convenient to incorporate into the synthetic gene of TPA a sequence that provides adequate spacing between the Shine-Dalgarno region and the initiation codon.

Proper selection of the sequence will minimize secondary structure that would otherwise interfere with transcription and translation, incorporate maximal codon usage (Grosjean H. and Fiers, W., Gene, 18:199-209, 1982); and satisfy the statistical biases found in the sequence around the ribosome binding site (Gold, L., et al., Ann. Rev. Microbiol., 35:365-403, 1981). The specific sequence for this invention is described herein by its actual sequence. However it is understood that alternative sequences could be used to optimize the expression of TPA analogs in different host cells.

E. Cloning the cDNA and Synthetic Portions of the TPA Gene.

Methods used to clone the cDNA and the synthetic portions of TPA for replication of the recombinant fragments coding for portions of TPA are standard procedures known in the art. The Maniatis manual containing methodology sufficient to conduct all subsequently described clonings. All clonings were done in transformed E. coli with any well characterized strain being useful. Strain HB101 is preferred unless otherwise stated.

Cloning vectors useful for transforming E. coli include pBR322 and pKC7.

F. Insertion of Unique Restriction Sites in the DNA-coding TPA.

The specific restriction sites chosen for the interdomain regions of TPA are illustrated in Charts 10 and 11. These are not the only restriction sites that would be useful in this invention. Restriction sites are chosen on the basis of their being unique in the TPA cDNA. Sites that will introduce minimal amino acid changes in the interdomain regions are preferred and those that are silent with respect to amino acid changes are most preferred (see Tables 1 and 2). Multiple sites having different reading frames within an interdomain are useful in order to ensure that the domains are in phase after ligation to other domains for purposes of expression. Duplicate restriction sites can be eliminated by simply digesting the site and ligating after making the ends blunt ended. The introduction of a unique site can be engineered in by chemical synthesis, commercially available linkers or by single site mutation.

The native TPA cDNA does not contain sites recognized by the following endonuclease restriction enzymes in addition to those sites engineered into the two prototype cDNA of TPA illustrated in Charts 10 and 11:

| ACC 1 | AFL 2 | AFL 3 | AHA 3 |
| ASU 2 | AVA 3 | AVR 2 | BCL 1 |
| BGL 1 | BSS H2 | HIND 3 | KPN 1 |
| MLU 1 | MST 2 | NAE 1 | NCO 1 |
| NDE 1 | NHE 1 | NOT 1 | NRU 1 |
| NSI 1 | PVU 1 | SAC 2 | SAL 1 |
| SAU 1 | SFI 1 | SNA 1 | SNA B1 |
| SPE 1 | XHO 1 | | |

G. Expression of TPA Analogs in E. coli.

To obtain high level expression of a cloned gene, e.g., the modified TPA gene, in a prokaryotic system, it is essential to construct expression vectors which contain, at the minimum, a strong promoter to direct mRNA transcription, a ribosome binding site for translational initiation and transcription terminator. Since the accumulation of large amounts of a gene product often inhibits cell growth and sometimes causes cell death, the promoter chosen to direct the synthesis of the product must be regulated in such a way that cell growth can be allowed to reach high densities before the induction of the promotor. Examples of regulatory regions suitable for this purpose are the promoter and operator region of the E. coli tryptophan biosynthetic pathway as described by Yanofsky, C., Kelley, R.L. and Horn, V., J. Bacteriol., 158:1018-1024 (1984) and the leftward promoter of phage lambda ($P_L$) as described by Herskowitz, I. and Hagen, D., Annu. Rev Genet., 14:399-445 (1980). The TPA produced in E. coli does not fold properly due to the large number of cysteine residues. The E. coli-produced TPA must first be denatured and then renatured. This can be accomplished by solubilizing the E. coli-produced TPA in guanidine HCl and reducing all the cystine residues with $\beta$-mercaptoethanol. The protein is then renatured either by slow dialysis or by gel filtration. US Patent No. 4,511,503.

H. Expression of TPA Analogs in Yeast.

The expression of heterologous proteins in yeast is well known. Methods in Yeast Genetics, Sherman, F., et al., Cold Spring Harbor Laboratory, (1982) is a well recognized work describing the various methods useful for producing TPA analogs in yeast.

For high level expression of a gene in yeast, it is essential to connect the gene to a strong promoter system as in the prokaryote and to also provide efficient transcription termination/polyadenylation sequences from a yeast gene. Examples of useful promoters include GAL1,10 (Johnston M., and Davis, R.W., Mol. and Cell. Biol., 4:1440-48, 1984), ADH2 (Russell, D., et al., J. Biol. Chem. 258:2674-2682, 1983), PHO5 (EMBOJ. 6:675-680, 1982) and MFα1. A multicopy plasmid with a selective marker such as Leu-2, URA-3, Trp-1, and His-3 is also desirable.

When cells are grown on glucose, GAL and ADH2 promoters are repressed, thus, allowing cells to be grown to a high density. Whereas the GAL promoters can be turned on by transferring cells to galactose medium, ADH2 will be turned on after all the glucose has been utilized by the cells. The PHO5 promotor can be switched on or off by manipulations of phosphate concentration in the medium. The MFα1 promoter in a host of the α mating-type is on all the time, but is off in diploids or cells with the a mating-type. It can, however, be regulated by raising or lowering temperature in hosts which have a ts mutation at one of the SIR loci. The effect of such a mutation at 35°C on an α type cell is to turn on the normally silent gene coding for the a mating-type. The expression of the silent a mating-type gene, in turn, turns off the MFα1 promoter. Lowering the temperature of growth to 27°C reverses the whole process and turns the a mating-type off and turns the MFα1 on (Herskowitz, I. & Oshima, Y. (1982) in The molecular biology of the yeast saccharomyces, (eds. Strathern, J.N., Jones, E.W., & Broach, J.R. Cold Spring Harbor Lab., Cold Spring Harbor, NY, pp 181-209).

The polyadenylation sequences are provided by the 3'-end sequences of any of the highly expressed genes, like ADH1, Mfα1, or TPI (Alber, T. and Kawasaki, G., J. of Mol. & Appl. Genet. 1:419-434, 1982).

A number of yeast expression plasmids like YEp6, YEp13, YEp24 can be used as vectors. A gene of interest such as TPA can be fused to any of the promoters mentioned above, and then ligated to the plasmids for expression in various yeast hosts. The above-mentioned plasmids have been fully described in the literature (Botstein, et al., Gene, 8:17-24, 1979; Broach, et al., Gene, 8:121-133, 1979).

Although the above-mentioned plasmids can be and have been used to express foreign genes in yeast, disclosed herein are vectors which allow significant flexibility with respect to insertion and/or excision of various components of the expression vectors. One such example is the vector pα1-ADHt illustrated in Chart 18.

Two procedures are used in transforming yeast cells. In one case, yeast cells are first converted into protoplasts using zymolyase, lyticase or glusulase, followed by addition of DNA and polyethylene glycol (PEG). The PEG-treated protoplasts are then regenerated in a 3% agar medium under selective conditions. Details of this procedures are given in the papers by J.D. Beggs, Nature (London), 275:104-109 (1978); and Hinnen, A., et al., Proc. Natl. Acad. Sci.-USA, 75:1929-1933 (1978). The second procedure does not involve removal of the cell wall. Instead the cells are treated with lithium-chloride or acetate and PEG and put on selective plates (Ito, H., et al., J. Bact., 153:163-168, 1983).

TPA analogs can be isolated by lysing the cells and applying standard protein isolation techniques to the lysates. TPA analogs can be detected by using Western blot techniques or radioimmunoassays.

I. Expression in Cell Cultures.

The TPA analog encoding DNA can be ligated to various expression vectors for use in transforming host cell cultures. The vectors all contain gene sequences to initiate transcription and translation of the TPA analogs that are compatible with the host cell to be transformed. When the host cell is a higher animal cell, e.g., a mammalian cell, the naturally occurring transcription and translation gene sequence of the TPA gene can be employed or the naturally occurring gene sequence can be employed along with a heterologous promoter sequence. In addition, the vectors preferably contain a marker to provide a phenotypic trait for selection of transformed host cells. Additionally a replicating vector might contain a replicon.

Illustrative of cell cultures useful for the production of TPA analogs are cells of insect or mammalian origin. Mammalian cell systems often will be in the form of monolayers of cells although mammalian cell suspensions may also be used. Illustrative examples of mammalian cell lines include VERO and HeLa cells, Chinese hamster ovary (CHO) cell lines, WI38, BHK, COS-7 or MDCK cell lines. Illustrative insect cell lines include Spodoptera frugiperda (fall armyworm) and Bombyx mori (silkworm).

As indicated above, the vector, e.g. a plasmid, which is used to transform the host cell, preferably contains gene sequences to initiate the transcription and translation of the TPA gene sequence. These sequences are referred to as expression control sequences. When the host cell is of insect or mammalian origin illustrative useful expression control sequences are obtained from the SV-40 promoter (Science, 222, 524-527, (1983)), the CMV I.E. promoter (Proc. Nat'l. Acad. Sci., 81:659-663, 1984) or the metallothionein promoter (Nature 296, 39-42 (1982)). As noted above, when the host cell is mammalian one may use the expression control sequences for the TPA gene but it is preferably to combine the TPA analog with a heterologous transcription initiation site. The plasmid or replicating or integrating DNA material containing the expression control sequences is cleaved using restriction enzymes and adjusted in size as necessary or desirable and ligated with cDNA coding for TPA analogs by means well known in the art.

As with yeast, when higher animal host cells are employed, polyadenylation or terminator sequences from known mammalian genes need to be incorporated into the vector. An example of a terminator sequence is the polyadenylation sequence from the bovine growth hormone gene.

Additionally, gene sequences to control replication in the host cell may be incorporated into the vector such as those found in bovine papilloma virus type-vectors. Saveria-Campo, M., "Bovine papilloma virus DNA: a eukaryotic cloning vector" in DNA Cloning Vol II a practical approach. Ed. D.M. Glover, IRL Press, Arlington, Virginia pages 213-238 (1985).

The host cells are competent or rendered competent for transformation by various means. There are several well-known methods of introducing DNA into animal cells. These include: calcium phosphate precipitation, fusion of the recipient cells with bacterial protoplasts containing the DNA, treatment of the recipient cells with liposomes containing the DNA, and microinjection of the DNA directly into the cells.

The transformed cells are grown up by means well known in the art, Biochemical Methods in Cell Culture and Virology, Kuchler, R. J., Dowden, Hutchinson and Ross, Inc., (1977), and the expressed TPA analogs are harvested from the cell medium in those systems where the protein is excreted from the host cell or from the cell suspension after disruption of the host cell system by, e.g., mechanical or enzymatic means which are well known in the art.

J. Evaluation of TPA Analogs.

TPA and its analogs can be evaluated in either of two ways. The relative ability to cleave plasminogen into plasmin can be assessed and the relative ability of inhibitors to prevent production of plasmin can be assessed. The following details describe the procedures for such evaluations.

Amidolytic assay. The functional activity of the TPA analogs is measured by using the coupled amidolytic assay described by Verheijen, J.H., et al., A simple, sensitive spectrophotometric assay for extrinsic (tissue-type) plasminogen activator applicable to measurements in plasma. Thromb. Haemostas., 48:266-269 (1982). Briefly, samples containing the TPA analogs are mixed with plasminogen and CNBr-digested fibrinogen fragments. The plasmin thus formed then cleaves an exogenously added chromogenic substrate, S-2251 (H-D-Valyl-L-leucyl-L-lysine-p-nitroanilide dihydrochloride; KABI, Stockholm, Sweden), generating a colored product that is monitored spectrophotometrically. The effect of protease inhibitors on the activity of the TPA analogs is assessed by using a modification of this assay described by Verheijen, J.H., et al., Evidence for the occurrence of a fast-acting inhibitor for tissue-type plasminogen activator in human plasma. Thromb. Haemostas., 51:392-395 (1984). The added inhibitor binds to the TPA, forming an irreversible complex and thereby preventing the TPA from activating the plasminogen. For example, purified native TPA is titrated with increasing amounts of an inhibitor-containing sample and the residual TPA activity is measured as outlined above. A standard curve is then developed by graphically representing the residual TPA activity as a function of the amount of inhibitor-containing sample added. In like manner, the TPA analogs are titrated with the inhibitor. The resulting curves are compared to that of the TPA standard. The degree of similarity between the curves relates directly to the susceptibility of the particular analog to inhibition.

Fibrin autography. The molecular weights of the TPA analogs are estimated by subjecting samples that contain the analogs to sodium dodecyl sulfate-polyacrylamide gel electrophoresis (SDS-PAGE) and then placing the acrylamide gel onto a plasminogen-containing fibrin indicator film. As the analog proteins diffuse from the acrylamide gel into the indicator film they convert the plasminogen into plasmin, which causes the formation of a clear zone of fibrinolysis in the otherwise opaque fibrin indicator. The appearance of this zone indicates the presence of an active TPA analog at a corresponding position in the acrylamide gel. This technique also is used to assess the interaction between TPA analogs and protease inhibitors. As mentioned above, this interaction results in the formation of an enzyme-inhibitor complex, which has a higher molecular weight than the analog itself. After SDS-PAGE, the complex exhibits residual TPA activity that can be visualized in the fibrin indicator film. The details of this technique were originally described by Granelli-Piperno, A. and E. Reich, A study of proteases and protease-inhibitor complexes in biological fluids. J. Exp. Med., 148:223-234 (1978). Assessment of the antigen of TPA analogs

Sandwich-type enzyme-linked immunosorbent assays (S-ELISAs). The antigen of TPA analogs is measured immunologically by two different S-ELISAs. The first employs goat polyclonal antibodies specific for human uterine TPA. The second uses a murine monoclonal antibody. This monoclonal antibody is specifically directed against an epitope required for the activity of TPA. The use of an antibody specific for the active-site domain of TPA provides an effective way of measuring the antigen of any TPA analog whose primary structure has been altered in a domain other than the active site. This assay is similar to one described by Korninger, C., et al., Sandwich ELISA for TPA antigen employing a monoclonal antibody. Thromb. Res., 41:527-535 (1986). The limit of sensitivity for both of the assays is approximately 1 ng of TPA per ml.

Western immunoblotting technique. The ability of the TPA analogs to form complexes with inhibitors also can be assessed by using SDS-PAGE and Western immunoblotting techniques as originally described by Towbin, H., et al., Electrophoretic transfer of proteins from polyacrylamide gels to nitrocellulose sheets: procedure and some applications, Proc. Natl. Acad. Sci. USA, 76:4350-4354 (1979). Briefly, the TPA is allowed to interact with the inhibitor, the mixture is subjected to SDS-PAGE, and the proteins are electrotransfered to nitrocellulose paper. Both uncomplexed, free TPA and TPA in complex with the inhibitor are detected by using purified polyclonal goat IgG specific for TPA and a rabbit antiserum directed against the goat IgG. The TPA-containing immunocomplexes are then visualized by using I[125]-labeled protein A and autoradiography.

DEFINITIONS

The term "cell culture" refers to the containment of growing cells derived from either a multicellular plant or animal which allows for the cells to remain viable outside the original plant or animal.

The term "domain" refers to a discrete continuous part of an amino acid sequence that can be equated with a particular function. With respect to TPA, the above cited references have defined the domain regions and Table 4 herein discloses the approximate locations of the mid points of the interdomain regions which lie between the domains.

The term "downstream" identifies sequences proceeding farther in the direction of expression; for example, the coding region is downstream from the initiation codon.

The term "interdomain" refers to the regions of a protein's amino acid sequence that lie between the domains. In this application the interdomain regions are plus or minus five amino acid residues from the mid points illustrated in Table 4. Thus the interdomain region between the finger and growth factor domains lies between and including amino acids 44 to 55; the interdomain between the growth factor and kringle 1 domains lies between and including amino acids 86 to 97; the interdomain between kringle 1 and kringle 2 domains lies between and including amino acids 169 to 180; the interdomain between kringle 2 and the active site lies between and including amino acids 257 to 268.

The term "maintained" refers to the stable presence of a plasmid within a transformed host wherein the plasmid is present as an autonomously replicating body or as an integrated portion of the host's genome.

The term "microorganism" includes both single cellular prokaryote and eukaryote organisms such as bacteria, actinomycetes and yeast.

The phrase "non-native endonuclease restriction sites" refers to endonuclease restriction sites that are not found at the equivalent position of the native cDNA sequence. These include both unique and nonunique sites.

The term "operon" is a complete unit of gene expression and regulation, including structural genes, regulator genes, and control regulation, including structural genes, regulator genes, and control elements in DNA recognized by regulator gene product.

The term "plasmid" refers to an autonomous self-replicating extrachromosomal circular DNA and includes both the expression and nonexpression types. Where a recombinant microorganism or cell culture is described as hosting an expression plasmid the term "expression plasmid" includes both extrachromosomal circular DNA and DNA that has been incorporated into the host chromosome(s).

The term "promoter" is a region of DNA involved in binding the RNA polymerase to initiate transcription.

The term "DNA sequence" refers to a single or double stranded DNA molecule comprised of nucleotide bases, adenosine, thymidine, cytosine and guanosine.

The term "suitable host" refers to a cell culture or microorganism that is compatible with a recombinant plasmid and will permit the plasmid to replicate, to be incorporated into its genome or to be expressed.

The term "upstream" identifies sequences proceeding in the opposite direction from expression; for example, the bacterial promoter is upstream from the transcription unit, the initiation codon is upstream from the coding region.

Conventions used to represent plasmids and fragments in Charts 1-27, though unique to this application, are meant to be synonymous with conventional representations of plasmids and their fragments. Unlike the conventional circular figures, the single line figures on the charts represent both circular and linear double-stranded DNA with initiation or transcription occurring from left to right (5' to 3'). Asterisks (*) represent the bridging of nucleotides to complete the circular form of the plasmids. Fragments do not have asterisk marks because they are linear pieces of double-stranded DNA. Endonuclease restriction sites are indicated above the line. Gene markers are indicated below the line. Bars appearing below the diagrams representing the plasmid or fragments are used to indicate the number of basepairs between two points on the DNA. The relative spacing between markers do not indicate actual distances but are only meant to indicate their relative positions on the illustrated DNA sequence.

PREPARATIONS

Preparation 1. The Vector - pSK4 - Charts 1-3.

Plasmid pSK4 is an expression vector capable of directing expression of heterologous proteins in E. coli. It has a strong, regulatable promoter to mediate transcription and a strong ribosome binding site for translation initiation. Specifically pSK4 uses the promoter and ribosome binding site (RBS) from the trpytophan (trp) operon. A unique ClaI site immediately downstream from the RBS is available for insertion of desirable genes such as TPA. (Chart 3).

To make plasmid pSK4, it is necessary to clone novel pTRZ1 (Chart 1) with pKC7, which is known (Chart 2). Rao, R.N. and Rogers, S.G., Gene, 7:79-82 (1979). Plasmid pTRZ1 contains a portion of the trp operon which includes the promoter/operator region, ribosome binding site, and a trpLE fusion ΔLE1413 as well as the structural genes for the galactosidase (lacZ) and lactose permease (lacY). Plasmid pKC7 is a derivative of pBR322 specifying resistance to ampicillin and kanamycin and having many unique restriction endonuclease sites.

The recombinant plasmid of pTRZ1 and pKC7 yields pSK3 having the trp promoter, RBS, and LE within

10

pKC7. Plasmid pSK3 is then modified to eliminate the fused peptide sequence trpLE to yield the general expression vector pSK4.

(1) Construction of pTRZ1 - Chart 1.

Plasmid pTRZ1 is a clone of known plasmids pMC1403 and pVV1.

Plasmid pMC1403 provides the lacZ gene of pTRZ1 minus the N-terminal eight amino acids and is fully described by Casadaban, M.J., et al., J.Bacteriol, 1980, 143: 971-980. Plasmid pMC1403 has three unique restriction enzyme cleavage sites. It is cut with EcoRI, dephosphorylated with bacterial alkaline phosphatase, and the ends are made blunt with Klenow fragment of E. coli DNA polymerase I. Plasmid pVV1 provides the trp promoter and operator sequence and is fully described by Nichols, B.P. and Yanofsky, C., 1983, Methods in Enzymology, eds. L. Grossman and K. Moldave, 101: 155-164, Academic Press, N.Y. Plasmid pVV1 contains the trp operon, with a 952 basepair deletion (ΔLE1413) fusing the trp leader sequence to the trpE gene forming trpLE. Plasmid PVV1 is cut with PvuII and BglII to yield fragment 2 (323 bp) which is isolated by preparative agarose gel electrophoresis. The BglII site is filled by Klenow enzyme. Fragment 2 is then ligated with pMC1403 to give pTRZ1.

(2) Construction of pSK3

The structural genes LacY and LacZ of pTRZ1 are unnecessary for the construction of pSK4 and their deletion will provide a smaller plasmid with more unique cloning sites. Additionally, the deletion of LacY will avoid the deleterious effects of over-production of a membrane-bound protein. As shown in Chart 2, the trp sequences (fragment 3) are excised from pTRZ1 by EcoRI/BamHI digestion, treated with alkaline phosphatase to minimize reinsertion, and then inserted into the EcoRI/BamHI region, fragment 4, of pKC7 which as a result of the digestion no longer specifies resistance to kanamycin. The clones having ampicillin resistance (AmpR) and kanamycin sensitivity (KanS) are screened for restriction fragments expected for the insertion of the trp promoter/operator sequence. This plasmid is called pSK3 and its equivalents are as described in Chart 2.

(3) Construction of pSK4

The trp promoter of pSK3 still retains the trpLE peptide which is unnecessary for the construction of a direct expression vector. The trpLE segment is excised by the following procedure. As described in Chart 3, the EcoRI/BamH fragment 5 carrying the trp sequences is cut from pSK3 and purified by electroelution from polyacrylamide gels. As shown, this fragment contains three TaqI sites, one of which is the promoter/operator region, (TaqI'') and one of which is between the ribosome binding site and the start of trpLE translation (TaqI'''). The fragment is partially digested by TaqI and the entire assortment of fragments are used in a ligation reaction with pBR322 previously cleaved with both EcoRI and ClaI.

TaqI recognizes T↓CGA (arrows denote point of strand scission) while ClaI recognizes AT↓CGAT. Since these two enzymes produce compatible cohesive ends, and the base 5' to the TaqI''' site between the ribosome binding site and trpLE is an A, (J. of Bact., 133:1457-1466) ligation of this TaqI end to the ClaI end regenerates the ClaI site. Note that this cloning scheme selects for single TaqI cuts and also ensures that transcription from the promoter will be in a clockwise direction. Clones displaying a 278 bp EcoRI/ClaI insert, and restriction patterns characteristic of the trp promoter are chosen and will be the equivalent of pSK4. The clones are useful for the expression of protein sequences capable of translation initiation when inserted at the ClaI site.

Preparation 2. Isolation of a Full Length cDNA Coding for Human Tissue Plasminogen Activator.

A. Materials and Methods

(1) Growth of Bowes Melanoma Cells

Bowes represents an established cell line derived from a human spontaneous melanoma and is a gift of Dr. Daniel Rifkin of the New York University School of Medicine. Cells are cultured in Dulbecco's Modified Eagle Medium (Gibco) supplemented with 10% fetal bovine serum (Gibco) and 50 $\mu$g/ml gentamycin (Sigma) in a humidified atmosphere at 37°, using 95% air/5% $CO_2$. Cells harvested for RNA preparation are grown to confluency in Falcon 150 $cm^2$ flasks.

(2) TPA mRNA Preparation

RNA from Bowes melanoma cells is isolated essentially as described by Lizardi, et al. Anal. Biochem., vol. 98, pp. 116-122 (1979).

DNA is polyA$^+$ enriched by passage over an oligodeoxythymidine-cellulose (OdT-cellulose-Collaborative Research) column as described by Aviv, H. et al., Proc. Nat. Acad. Sci., vol. 69, pp. 1408-12 (1972). Typical yield from 10-150 cm$^2$ flasks twice selected on separated OdT columns is approximately 50 $\mu$g polyA$^+$ mRNA.

The polyA$^+$ mRNA is fractionated by centrifugation through 15-30% linear sucrose gradients. PolyA$^+$ mRNA (~300 $\mu$g) dissolved in 200-400 $\mu$l of a sterile distilled $H_2O$ is loaded onto a gradient and centrifuged in a Beckman SW41 Ti rotor at 35,000 RPM for 18 hours, 0.5 ml fractions are collected, using the Buchler Auto Densi-Flow Model IIC to harvest from top to bottom, connected to a Gilson Micro fractionator (Model FC80-K). Aliquots from each fraction are injected into Xenopus oocytes and translation products are assayed for plasminogen activator activity by the casein-agar plate method analogous to that described by Miskin, R. et al., Nuc. Acids Res. vol. 9, pp. 3355-63. TPA activity is rendered by mRNA migrating at about 19S.

Specifically, the tissue plasminogen activator TPA is assayed by observing the conversion of plasminogen to plasmin, a non-specific protease, which digests casein. The assay is performed as follows. 0.5 ml of 8% casein, boiled, is mixed with 1 ml 2 x MBS and heated to 50°C. 3% agarose is melted and cooled to 50°C. 0.5 ml of the 3% agarose is added to the casein/MBS and mixed by pipetting. 100$\mu$l of 1 mg/ml human plasminogen is added (final concentration is 50 $\mu$g/ml) with mixing and the material is poured into a 3.5 cm diameter plastic petri dish. The agarose solution is cooled to room temperature (about 30 minutes). 2 mm diameter holes in the agarose are cut with Biorad cutter gel punches. The holes are made just before use and the wells are filled with MBS. One or two oocytes which have been injected with TPA RNA at least 6 hours prior to assay are added to each well. Incubation is in a humidified dish for 12-24 hours. Clear zones of casein hydrolysis are readily observed.

Fractions enriched for TPA mRNA are precipitated by addition of 2 volumes of ethanol and incubating on dry ice for 1 hour. Precipitates are collected by centrifugation at room temperature in an Eppendorf table-top microfuge, dissolved in sterile, distilled $H_2O$, pooled, and reprecipitated. This t-PA enriched polyA$^+$ mRNA is used to generate cDNA for cloning.

(3) cDNA Synthesis

All of these reactions are assembled on ice.

(A) First Strand Synthesis using OdT$_{12-18}$ Primer

10 $\mu$g of enriched, polyA + selected Bowes mRNA (in $\mu$l $H_2O$ is incubated at room temperature for 10' in the presence of 10 mM MeHgOH (Alfa) to aid in the unfolding of secondary structure. Subsequently, the following components are added; $\beta$-mercaptoethanol to 28 mM; RNasin (Biotec Inc) 1 unit/$\mu$l final reaction vol; 75 mM Tris base pH 8.3; 6 mM $MgCl_2$; 50 mM KCl; 10 $\mu$g oligodeoxythymidine (OdT$_{12-18}$-Collaborative Research); 100 $\mu$Ci $\alpha$-$^{32}$P-dCTP (Amersham); dGTP, dTTP, and dATP to 500 $\mu$m each; dCTP to 250 $\mu$m (all nucleotides from P. L. Biochemicals, dissolved to 20 mM in 10 mM Tris base pH 8 and neutralized with 1.0 M NaOH) and reverse transcriptase (Life Sciences Inc.) 1 unit/$\mu$g input RNA, in a final reaction volume of 50 $\mu$l. The reaction is allowed to incubate for 60 minutes at 42°C.

(B) First Strand Synthesis using Specific 15-mer oligonucleotides as Primers (see Panabieres, F. et al., Gene, vol. 19, pp. 321-6 (1982) for generally analogous procedures).

The obtaining of a complete cDNA strand coding for TPA from mRNA primed at the 3' polyA tail is not always possible using the above procedure. By priming with a sequence upstream from the polyA tail one is more likely to obtain a sequence of 5' TPA-coding cDNA that can be joined with an incomplete cDNA that was initiated at the polyA tail sequence.

Three pentadecamers are preferred for use as either probes or as primers for cDNA synthesis. Their sequences are:

|  | 5' |  |  |  |  | complementary to 3' TPA positions; |
|---|---|---|---|---|---|---|
| 1. | TCA | CGG | TCG | CAT | GTT | 1759-1773 |
| 2. | GGG | GTT | TGA | GTC | TCG | 634-648 |
| 3. | CCC | ATC | AGG | ATT | CCG | 886-900 |

They are synthesized and purified by methods previously described.

Twenty-Five $\mu$g of polyA + selected RNA is incubated with 1 $\mu$g of primer (5-10-fold picomolar excess of primer to template) and 1.5 mM EDTA in 58 $\mu$l at 90° C. The mixture is allowed to equilibrate to room temperature slowly by immersion in a 5 ml water bath heated initially to 90°. After annealing the pentadecamer to the RNA template the following reagents are added: dithiothreitol (DTT) to 2 mM; RNasin to 1 unit/$\mu$l final reaction volume; 100 mM Tris base, pH 8.3; 11 mM $MgCl_2$; 50 mM KCl; 100 $\mu$Ci $\alpha$-$^{32}$P-dCTP; 500 $\mu$m each of dGTP, dTTP, and dATP; 250 $\mu$M dCTP; reverse transcriptase (Life Sciences, Inc.) to 1 unit/$\mu$g RNA. Incubation is at 20° C for 3 hours at which point an equal amount of additional reverse transcriptase is added and the reaction is allowed to continue at 50° for 30 minutes. The reaction is terminated by phenol extraction and the RNA template is removed by alkaline hydrolysis as fully described in Maniatis.

(C) Synthesis of the Second Strand.

The second strand is synthesized in a reaction consisting of 40 mM $KPO_4$ (K-phosphate) pH 7.5; 6.6 mM $MgCl_2$; 1 mM DTT; 500 $\mu$m each of dGTP, dTTP, dATP; 250 $\mu$m dCTP; 100 $\mu$Ci $^3$H-dCTP (Amersham) and 1 unit/$\mu$g input RNA of DNA polymerase Klenow fragment (BRL) in a final volume of 100 $\mu$l. The reaction is incubated at 15° C for 4 hours and may be followed by monitoring the incorporation of $^3$H-dCTP. The reaction is terminated by phenol extraction, and double stranded cDNA precipitated as described above for first strand synthesis with the exception that $NH_4Ac$ is added to 2.5 M (rather than NaCl to 0.3 M) for the first ethanol precipitation.

$S_1$ is used to remove hairpin structures. The $S_1$ reactions are carried out according to Maniatis. Successful elimination of hairpins is determined by an increase of TCA-soluble counts. The reaction is terminated by phenol extraction omitting the precipitations. The aqueous phases are pooled and loaded directly onto sucrose gradients identical to those used for fractionation of polyA$^+$ mRNA. Fractions are collected and assayed by dissolving 5 $\mu$l aliquots into 10 ml of Aquafluor scintillation fluid (New England Nuclear) and measuring both $^{32}$P and $^3$H. cDNA from relevant fractions are precipitated by addition of 2 volumes of ethanol and incubating on dry ice for 30 minutes. The precipitates are pelleted by centrifugation for 15' at room temperature in an Eppendorf microfuge. Pellets are dissolved in sterile 0.3 M NaCl, pooled and reprecipitated. The precipitates are pelleted and dried.

(D) Tailing and Annealing of the cDNA to Vector DNA.

Homopolymer tracts of dCTP are enzymatically added to the 3' termini of the cDNA molecules according to the methods described by Maniatis. Ideally, 10-30 residues of dCTP should be added to maximize cloning efficiency.

Vector DNA (prepared by digesting pBR322 to completion with PstI and adding homopolymer tracts of dGTP residues) is commercially available from New England Nuclear. The vector DNA can also be made according to the methods described by Maniatis. The procedure for annealing cDNA with vector DNA is also described by Maniatis. Briefly, tailed cDNA is mixed with vector in a 1:1 molar ratio in a 50 $\mu$l reaction containing 10 mM Tris pH 7.4; 0.4 M NaCl; 1 mM EDTA. Final DNA concentrations varied between 20-60 $\mu$g/ml. Annealing is accomplished by either; 1) following a defined regimen of incubations consisting of 65°/10'; 42°/60'; 37°/2 hours, and then room temperature for 2 hours, or 2) incubation at 65°/10' shutting off the water bath and allowing it to slowly equilibrate to room temperature overnight.

(E) Cloning and Sequence Confirmation of Full Length TPA cDNA. Charts 4-5.

The cDNA containing vectors are introduced into E. coli using transformation procedures already described. The bacteria are screened in situ using the hybridization procedures described earlier.

The colony hybridizations described here use the pentadecamers also described above as probes. For use as a hybridization probe one $\mu$g of 15-mer is phosphorylated in a 50 $\mu$l reaction volume consisting of 70 mM Tris-base (pH 7.6), 100 mM KCl; 10 mM $MgCl_2$, 5 mM dithiothreitol, and 50 $\mu$Ci $\gamma^{32}$P dATP (P. L. Biochemicals), and 1 U $T_4$ polynucleotide kinase (New England Biolabs). Incubation is at 37° for 60 minutes. In this fashion, the 15-mer can be labelled to specific activity of $1 \times 10^8$ cpm per $\mu$g.

Clones exhibiting complementary sequences to the probes are selected for secondary screening using PstI restriction analysis of the clones to determine if the digestion products are consistent with the PstI restriction map which can be obtained from the sequence given in Table 1 or Pennica et al., "Cloning and Expression of Human Tissue-type Plasminogen Activator cDNA in E. coli.", Nature, vol. 301, pp. 214-21 (20 Jan. 1983). The desirable clones will have a large portion of the 3' portion of the TPA cDNA. Digestion yields 4 fragments: the original pBR322 vector, an 1150 bp fragment, an 80 bp fragment and a 78 bp fragment. These results suggest an insert spanning about 1300 bp of the gene's 3'-end (nucleotides 1250-2550). To confirm this assumption, the clone is double digested with PstI and DdeI. The latter enzyme should cut the 1150 bp fragment into a 926 bp fragment and a 229 bp fragment while leaving the 78 or 80 bp fragments intact. The results of such a double digest will support the conclusion that the clone does in fact represent the 3'-end of the TPA gene.

As final proof, a mini-preparation of DNA is isolated from the clone and is sequenced by dideoxy chain termination. Minipreps of plasmid DNA are prepared as described in the General Methods section. Dideoxy sequencing is carried out as described in the General Methods section using Pentadecamer #1 as a primer in a 20:1 molar excess over template. The sequence data derived from this clone, designated pTPA H, is identical to sequence data presented by Pennica et al., Nature, vol. 301, pp. 214-21 (1983) for human tissue plasminogen activator (Chart 4).

From here the focus is on isolation of the 5'-end of the gene. To do this, cDNA is synthesized from twice-selected polyA + mRNA by primer extending from pentadecamer #1 rather than from $OdT_{12-18}$. The advantage afforded by this approach is twofold. First, because priming is specific for TPA, a higher percentage of the cDNA made should be represented by TPA specific sequences. Secondly, priming is some 800 bp 5' of the polyA tail, the site utilized by $OdT_{12-18}$ to prime first strand synthesis. This almost assures that the cDNA derived from this approach will include transcripts extending further 5' than pTPA H.

Transformation efficiency with this TPA-primed, cDNA is $2.5 \times 10^5$ transformants per $\mu$g of cDNA. Two banks totaling 25,000 transformants are generated. The concept of "gene-walking" is applied to purposely bias the screening. The pTPA H 5'-terminal 80 bp PstI fragment is gel isolated and used to probe 28,000 colonies derived from the primer extended banks. This specifically selects for clones extending at least as far 5' as pTPA H, and as a result of the primer extension approach, clones containing additional 5' sequences as well.

The screening by in situ hybridization yields a number of positives. The longest TPA insert is detected in secondary screening with PstI and DdeI. In this invention the clone, designated pTPA80-1, included an insert spanning nucleotides 550-1600 (chart 4).

The restriction data make it possible to determine fairly accurately (+/- ~10%) the position of the 3' terminus. If priming begins at nucleotide 1759, and pTPA80-1 has a 3' terminus at about position 1600, then a loss of approximately 160 basepairs occurs at some point during the cloning. The $S_1$ endonuclease is the most likely cause of the loss.

The orientation of pTPAH and pTPA80-1 are determined. Plasmids pTPAH and pTPH80-1 are cut with SacI and PvuI to yield fragments 7 (1251 bp) and 8 (5.1 kb) respectively which are gel isolated. The fragments are treated with bacterial alkaline phosphatase and ligated using T4 polymerase to yield pTPA3'cDNA (6.3 kb) having positions 550 to 2230 bases of the TPA cDNA. The new construct is verified by PstI digestion.

Since pTPA80-1 represents the longest TPA insert amongst the positives detected in the first primer extended banks, a second oligonucleotide complimentary to the coding sequence of amino acid residues 233-237 (nucleotides 886-900; 15-mer #2) is used to generate another cDNA library to complete the 5' end. This particular area of the gene is chosen so that should $S_1$ remove even as much as 200 bp from the 3' terminus of the transcript, sufficient overlap with pTPA3'cDNA would still exist to allow for assembly of the fragments.

The cDNA containing the 5' bases was ligated to PstI cleaved pBR322 and transformed in E. coli as described above. Transformation efficiency with this cDNA is on the order of $3.6 \times 10^4$ transformants/$\mu$g cDNA. Colonies from this library are screened with an oligonucleotide complimentary to coding sequence extending from nucleotides 645-660 (15-mer #3) and unambiguous positives are selected. Secondary

14

screening is accomplished by PstI and BglII digestion and one clone, designated pTPA5'cDNA was shown to contain the remaining 5' sequences 1-750. Again, the effects of $S_1$ on insert length are apparent with pTPA5'cDNA. While priming is initiated at nucleotide 886, pTPA5'cDNA extends only to about nucleotide 750; our work showed a loss of about 136 bp.

With the entire TPA sequence available on two clones (pTPA3'cDNA and pTPA5'cDNA) the final task of assembly remains. The strategy is depicted in Chart 5.

Plasmid pTPA5'cDNA is cut with TaqI to yield fragment 9 (2194 bp). One TaqI site within the TPA sequence of pTPA5'cDNA (position 634) is highly enzyme resistant. Fragment 9 is cut with BglII and HgaI to yield fragment 10 (405 kb) having bases 188 to 593 of TPA cDNA representing the mature portion of the protein.

The middle portion of the TPA cDNA is obtained by first cutting pTPA3'cDNA with PuvII and EcoRI isolating fragment 11 (1748 bp). Fragment 11 is then cut with HgaI to yield fragment 12 (209 bp) containing bases 594 to 802.

To obtain the 3' portion of the cDNA, pTPA3'cDNA is digested with PvuI and the 6.3 kb fragment is isolated, treated with T4 polymerase, and partially digested with EcoRI to yield fragment 13 (1871 bp) containing bases 802 to 2230.

The ligation of the three parts of the cDNA involves the use of pKC7 which is publicly available and fully described in Rao, R.N., and Rogers, S.G., Plasmid pKC7: a vector containing ten restriction sites suitable for cloning DNA segments, Gene 7:79:82 (1979). Plasmid pKC7 is digested with BglII and SmaI and treated with bacterial alkaline phosphatase to yield fragment 14 (4.8 kb) which is gel isolated.

Fragments 10,12,13 and 14 are ligated in a single ligation pool. One noteworthy aspect of this strategy is the means by which the need for alkaline phosphatase treatment of the internal fragment is obviated.

Typically, multipiece ($\geqq$ 4 fragments) ligations produce the desired construction with very low efficiency due to concatemerization of the fragments. Treatment with alkaline phosphatase in the appropriate fashion minimizes this problem, resulting in a dramatic increase in efficiency of correct, multipiece assembly. In assembling the TPA fragments, we took advantage of the unique way in which the restriction endonuclease, HgaI, cuts DNA. The recognition (binding) sequence of the enzyme is GACGC but the enzyme cuts at a point beyond that recognition sequence. As a result, fragments cut with HgaI will ligate only with their originally contiguous counterpart. Self-ligation promoted by the HgaI overhang cannot happen. The 4-piece TPA ligation contains two fragments bearing an HgaI terminus. That in addition to treatment of the vector with alkaline phosphatase (to minimize self-closure) results in most of the transformants bearing the correctly assembled TPA gene. Correct assembly is verified by the restriction digests. The plasmid containing the complete cDNA sequence for TPA is designated pTPAcDNA (7.4 kb).

EXAMPLES

Example 1. Preparation of synthetic oligonucleotides.

Eighty-seven separate oligonucleotides (Table 3) were synthesized according to the procedures described earlier. The described strategy will assemble the 1084 basepairs of synthetic DNA between the BglII site at position 187 and the EcoRI site at position 1287 into pTPA-B1,2,3 (Chart 9). The specific restriction sites chosen for insertion into the interdomain regions are depicted in Charts 10 and 11. Each block is recombined with a suitable cloning vector for replication in E. coli and for verifying the sequence. All ligation and cloning methodology used is as described by Maniatis et al. supra. All cloned sequences are sequenced using the Sanger dideoxy sequencing technique to verify the sequence.

Block 1. Oligonucleotides P1-P4, P8-P11 are annealed and ligated to form a double stranded segment which is ligated to a second segment comprised of oligonucleotides P5-P7 and P12-P14. The resulting block 1 contains the sequence coding for the finger domain. Block 1 is cloned into the BglII and SphI sites of pSK4 described in Preparation 1.

Block 2. Oligonucleotides P15-P18 and P19-P23 which contain the sequence coding for the growth factor domain are ligated together in a single step annealing and ligation to form block 2. and cloned into the SphI and ClaI site of pBR322.

Block 3. Oligonucleotides P24-P28, P34-P38 are annealed and ligated to form a double stranded segment which is ligated to a second segment comprised of oligonucleotides P29-P33 and P39-P43. The resulting block 3 contains the sequence coding for the kringle 1 domain. Block 3 is cloned into the ClaI and BamHI sites of pBR322.

Block 4. Oligonucleotides P44-P47 and P67-P70; P48-P51 and P71-P74; P52, P53, P92, P93 and, P75, P76, P94, P95; P57-P61 and P80-P84; and P62-P66 and P85-P89 are each annealed in 5 separate tubes into double stranded segments which are then pooled and ligated to form block 4. Block 4 contains the DNA sequence coding for the kringle 2 domain. Block 4 is cloned into the BamHI and EcoRI sites of pBR322.

Example 2. The assembly of blocks 1-4 with the active site of the TPA cDNA. Charts 6-11.

The following example provides a summary of the steps needed to assemble the various synthetic blocks of Example 1 into a gene capable of encoding for a biologically active TPA. Two prototype genes are described. The plasmid pTPA-B1,2,3,4(a) has a gene encoding TPA which has no artificially-introduced endonuclease sites between the kringle 2 domain and the active site. Plasmid pTPA-B,1,2,3,4 has a gene encoding TPA which does have artificially-introduced endonuclease restrition sites between kringle 2 and the active site.

A. Construction of pTPA-B1 - Chart 6.

Plasmid pSK4 is digested with ClaI and SphI and the large 4.1 kb fragment 15 is isolated and ligated to the Block 1 fragment using a ClaI/XbaI linker. The ligation mixture is transformed into HB101 using the calcium chloride transformation procedure described by Maniatis. The transformants are screened with nick translated Block 1 fragments using the nick translation procedure described by Maniatis et al. Transformants hybridizing to the probe are then sequenced using the Sanger dideoxy sequencing technique to verify the correct sequence and orientation. This new transformant is denoted pTPA-B1 (4.25 kb).

B. Construction of pTPA-B1,2 - Chart 7.

Plasmid pTPA-B1 is digested with EcoRI and SphI and fragment 16 (450 bp) is isolated. Plasmid pBR322 is digested EcoRI and ClaI and the large fragment 17 (4.35 kb) is gel isolated. Fragments 16 and 17 are then ligated to the synthetic SphI/ClaI Block 2. The ligation mixture is transformed into HB101 and the transformants screened with nick translated Block 2. Transformants hybridizing to the probe are sequenced to confirm that they contain Block 2 in its proper orientation. This construction is designated pTPA-B 1,2 (4.8 kb).

C. Construction of pTPA-B 1,2(a) - Chart 8.

Plasmid pTPA-B 1,2(a) contains HindIII and BglII restriction sites upstream from the finger and growth factor domains. Plasmid pTPA-B 1,2 is digested with XbaI and EcoRI and the large 4.5 kb fragment 18 is gel isolated and ligated to an oligonucleotide linker containing a HindIII and BglII site. The ligation mixture is transformed into HB101 and transformants screened for the presence of the HindIII/BglII sites. The new construction is designated pTPA-B 1,2(a) (4.5 kb).

D. Construction of pTPA-B 1,2,3 - Chart 9.

Plasmid pTPA-B 1,2(a) is digested with ClaI and BamHI and the large 4.0 kb fragment 19 is isolated. This fragment is ligated to the synthetic ClaI/BamHI kringle 1 region comprising block 3 (270 bp) and transformed into HB101. The transformants were screened with nick translated block 3. Transformants hybridizing to Block 3 were sequenced to confirm the sequence and orientation. This new construction is designated pTPA-B 1,2,3 (4.3 kb).

E. Construction of pTPA-B 1,2,3,4a - Chart 10

This plasmid contains an entire prototype TPA gene encoding for a TPA analog having enzymatic activity. There are in this gene no changes to the interdomain region between kringle 2 and the active site. Construction of pTPA-B 1,2,3,4a (4.2 kb) requires several steps. Plasmid pTPAcDNA (Chart 5) is cut with ScaI and fragment 20 (6.0 kb) encoding for the 3' end of the TPA gene is gel isolated. Plasmid pTPA-B 1,2,3 (Chart 9) is cut with ScaI and BamHI to obtain fragment 21 (1100 bp) containing the Finger, Growth Factor and kringle 1 domains. A third fragment 4a (230 bp) is obtained by cutting block 4 with BamHI and ScaI. The three fragments are ligated using T4 ligase to obtain pTPA-B 1,2,3,4a. The TPA gene is cut out of pBr322 using HindIII and AatII to yield a 2.2 kb fragment which is subcloned into pUC-19. Plasmid puC-19 is commercially available from a variety of sources and contains a selection of restriction sites in its DNA sequence which makes TPA domain manipulation less cumbersome.

F. Construction of pTPA-B 1,2,3,4 - Chart 11

This plasmid contains an entire prototype TPA gene encoding for a enzymatically active TPA analog. In this analog all 4 domains and the active site are present with unique restriction sites engineered into all the interdomain regions including the region between kringle 2 and the active site. The plasmid is constructed by first cutting pTPA-B 1,2,3,4a with EcoRI and BamHI and isolating the large 3.7 kb fragment containing the Finger, Growth Factor and kringle 1 domains. The synthetically created block 4 is obtained from its clone through a digestion with BamHI and partial digestion with EcoRI to yield intact block 4 having 560 kb. The two fragments are ligated using T4 ligase to obtain pTPA-B 1,2,3,4.

Plasmid pTPA-B 1,2,3,4a and pTPA-B 1,2,3,4 can be used to construct a variety of synthetic TPA analogs.

Example 3. Construction of TPA analogs.

A. Construction of pFK2A (deletion of growth factor and kringle 1 domain).

Plasmid pTPA-B 1,2,3,4 is digested with HpaI. The large 6.5 kb fragment is isolated and religated to form pFK2A. The plasmid is transformed into HB101 or some other suitable host and screened for correct orientation and sequence.

B. Construction of pFK2K2A - Chart 12 (deletion of growth factor and kringle 1 and duplication of kringle 2).

Plasmid pTPA-B 1,2,3,4 (Chart 11) is digested with HpaI to obtain fragment 22 (6.5 kb) which is isolated. A second sample of pTPA-B 1,2,3,4 is digested with HpaI and MstI and the resulting 560 bp fragment 23 containing the kringle 2 domain is isolated. The HpaI digested FK2A fragment is blunt end ligated to the HpaI/MstI fragment to yield pFK2K2A (6.5 kb). Plasmid pFK2K2A is then transformed into HB101 and screened for correct orientation and correct sequence.

C. Expression in E. coli Plasmid pTPAExpl - Chart 13.

Expression of TPA analogs can be accomplished in E coli by using the expression plasmid pTPA-B1,2 illustrated in chart 7, pTPA-B1 illustrated in Chart 6, or their equivalents. Any of the analogs can be placed within the XbaI and BamHI sites for expression. To express pFK2K2A in E. coli, pTPA-B 1,2 (Chart 7) and pFK2K2A (Chart 12) are both cut with XbaI and BamHI and fragments 24 (4.2 kb) and 25 (2.0 kb) are gel isolated. Fragments 24 and 25 are ligated to form expression plasmid pTPAExpl (6.2 kb).

Plasmid pTPAExpl contains a Trp promoter and operator and expression is constitutive. Culturing of E. coli is according to Maniatis (Supra). The E. coli cultures will not produce active TPA. The TPA produced by E. coli must be refolded into the native state. By following known cysteine reshuffling procedures one can obtain active TPA. US Pat. No. 4,511,502.

Example 4. Expression in Eukaryotes.

A. Expression of Human TPA in Yeast.

This example illustrates the construction of yeast expression plasmid pα1-FK2K2A having a MFα1 promotor and pre-pro sequences. Culturing conditions and preferred host strains are also provided for the expression of TPA analogs in yeast.

(1) Construction of pα1-ADHt, a Yeast Expression Vector.

Plasmid, pα1-ADHt, is a multi-purpose expression vector for yeast. Restriction sites at convenient points have been engineered into its structure and control under the MFα1 promoter is generally compatible with high levels of expression. The following steps describe the construction of pα1-ADHt. (Charts 14-18)

a. Construction of pYRep3'B-Chart 14.

Plasmid pYRep3'B is constructed by placing the REP III and replication origin sequences of the 2μ plasmid into the SmaI site of the URA3 gene of pYIP31 to create a convenient cassette. Both plasmids, 2μ and pYIP31, are publicly available and fully described in the literature. The 2μ plasmid was fully described by J.R. Broach in Molecular Biology of the Yeast Saccharomyces cerevisiae [Life cycle and inheritance], pp.445-470 and the RepIII region is described in Cell 34:95-104 (1983) and Cell, 35:487-493 (1983). Plasmid pYIP31 was described in Gene, 18:17-24 (1979).

Plasmid pYIP31 is first cut with SmaI and treated with bacterial alkaline phosphatase to yield fragment 26 (5.4 kb) which is isolated. The 2μ plasmid of yeast is cut with PstI and XbaI, filled with Klenow and fragment 27 (1.3 kb) is isolated. Fragments 26 and 27 are ligated using T4 DNA ligase to obtain pYRep3'B (6.7 kb).

b. Construction of pADHt-Charts 15-16.

Plasmid pADHt (3.86 kb) is useful because it provides useful restriction sites to assemble a yeast expression vector.

The starting plasmid for construction of pADHt is pGG400 (4.0kb). Plasmid pGG400 is constructed from plasmids pBR322 and pML21 which are readily available to the public. J. Bacter., 126:447-453 (1976). Specifically, the gene of pBR322 coding for tetracycline resistance is replaced with a section of pML21 coding for kanamycin resistance. Plasmid pBR322 is first cut with EcoRI and PvuII and the EcoRI overhang filled in with Klenow enzyme and isolated from an agarose gel to obtain fragment 28 (2.3 kb). Plasmid pML21 is cut with PuvII to yield fragment 29 (1.7 kb) carrying the gene for resistance to kanamycin. The ligation of a PuvII cut site with a filled EcoRI site will regenerate the EcoRI site after ligation. The ligation of fragments 28 and 29 yields pGG400 which is used to construct pADHt (Chart 15).

Plasmid pADHt is constructed by cutting pGG400 with PvuII and XhoI and treating with Klenow enzyme to obtain fragment 30 (3.5 kb) which is isolated. The 3' end of yeast alcohol dehydrogenase I (ADHI) is obtained from pADHBC by first cutting with HincII and BamHI, treating with T4 polymerase and isolating fragment 31 (0.36 kb). Plasmid pADHBC is publicly available and fully described in J. Biol. Chem. 257:3018-1025 (1982). Fragments 30 and 31 are ligated to obtain pADHt among others and transformed into E. coli. Those transformants carrying pADHt will have regenerated BamHI and XhoI sites in their plasmids. These transformants are selected and the authenticity of the cloned ADHI 3'-end is confirmed by restriction enzyme analysis and by sequencing (Chart 16).

c. Construction of pADHt-REPIII - Chart 17.

Plasmid pADHt-REPIII (6.2 kb) is a construction of pADHt and pYRep31B in which the URA3-REPIII-ori cassette is placed into pADHt to facilitate expression and replication in yeast. Plasmid pADHt-REPIII is useful for construction of expression vectors in yeast because the EcoRI-HindIII or EcoRI-SmaI fragments can be replaced with a desired DNA fragment carrying appropriate yeast promoters or it can be used to provide a SalI-XhoI fragment carrying a replication origin, a selectable marker and the 3' end of the yeast URA3 gene.

Plasmid pADHt is modified by cutting with BamHI, filling the ends with Klenow enzyme and inserting an 8-mer SalI linker into the filled BamHI site. Plasmid pADHt (modified) is cut with SphI and the ends are filled with T4 polymerase to yield fragment 32 (3.8 kb). Plasmid pYRep31B is cut with HindIII and fragment 33 (2.4 kb) is isolated containing the 3' end of URA3 and the 2μ RepIII region which is thought to increase stability and the origin of replication. Fragments 32 and 33 are ligated and the E. coli transformants having plasmids with an orientation of the URA3 gene allowing for a clockwise transcription are screened to obtain pADHt-REPIII.

d. Construction of pα1-ADHt - Chart 18.

Construction of pα1-ADHt (6.5 kb) is completed by replacing the EcoRI-HindIII fragment of pADHt-REPIII with the EcoRI-HindIII fragment from pα1 containing the promoter and pre-pro sequence of the MFα1 gene. Plasmid pα1 is fully described by A. Singh. et al., Nucleic Acid Research, 11:4049-63 (1983) and J. Kurjan, et al., Cell, 30:933-943 (1983). Plasmid pADHt-REPIII is first cut with EcoRI and HindIII to yield fragment 34 (5.3 kb) which is isolated. Plasmid pα1 is also cut with EcoRI and HindIII to yield fragment 35 (1.2 kb) which is isolated. Fragments 34 and 35 are then ligated using T4 DNA ligase to obtain pα1-ADHt.

(2) Construction of pα1-FK2K2A, Insertion of the TPA Analog cDNA into a Yeast Expression Vector - Chart

18

19.

The preferred expression vector for the production of TPA analogs in yeast is designated pα1-FK2K2A having an MFα1 promoter. Plasmid pFK2K2A (Chart 12) is cut with BglII to yield fragment 36 (2.0 kb) which is gel isolated. Plasmid pα1-ADHt is cut with HindIII and XhoI to yield fragment 37 (5.6 kb) and provides the transcription, the polyadenylation and translational sites. The system described herein will terminate downstream from the mature TPA protein and the resulting product will have a few additional amino acids of yeast origin. To terminate translation at the precise end of the TPA gene, one can provide a termination codon.

To ensure that the analog is in phase with the reading frame of the MFα pre-pro sequences, the 5'- and 3'-ends of the analogs are unchanged. The BglII site at the 5'-end can be manipulated either by inserting another linker or by a combination of Klenow (Pol I) and Mung Bean nuclease to create a HindIII site while maintaining the reading frame-in phase with the MFα1 'pre-pro' sequences. For this example the BglII site of fragment 37 is partially filled using Klenow with adenosine and guanosine. The partially filled site is then made blunt with Mung Bean nuclease and ligated to the HindIII site of pα1-ADHt. Fragments 36 and 37 (with the partially filled in end) are ligated to yield pFK2K2A (7.6 kb). Other analog TPA genes can also be inserted blunt-ended in the expression vector as long as the reading frame is maintained in phase with the yeast sequences.

3) Expression of TPA Analog from the Plasmid pα1-FK2K2A in Yeast.

Yeast strain YNN227 (α,ura3-52 trp1-289) are transformed with pα1-FK2K2A. The transformants are grown in glucose minimal medium (2% glucose, 0.7% yeast nitrogen base, 1% casamino acids, 40μg/ml adenine, 50μg/ml tryptophan) for 10 hours. Cells are then pelleted out by centrifugation. The cells are lysed by vortexing with glass beads. Quantities of TPA can be detected using Western Blotting techniques or radioimmunoassay or the casein enzymatic assay in agar described earlier for xenopus oocytes.

TPA can be isolated from yeast cells by first lysing the cells with 0.5 mm glass beads followed by purification on an affinity column. Standard protein purification schemes can be applied to affect further purification.

B. Expression of TPA Analogs in Chinese Hamster Ovary Cells - Charts 20-22.

The following example illustrates the preferred method for expressing pFK2K2A in Chinese hamster ovary (CHO) cells. In summary there is disclosed herein, a shuttle vector pSVCOW7 which replicates in both CHO and E. coli cells utilizing ampicillin resistance and dihydrofolate reductase genes as markers in E. coli and CHO cells respectively. Plasmid pSVCOW7 also provides the polyadenylation sequence from bovine growth home which is necessary for expression in CHO cells. Plasmid pSVCOW7 is first cleaved and a viral promoter and the TPA analog are inserted. Transformation culture conditions and extraction procedures for TPA expressed in CHO cells are also described below.

(1) Construction of pSVCOW7 - Chart 20

The starting plasmid pSV2dhfr (available from the American Type Culture Collection or prepared according to the procedure of S. Subramani, et al., "Expression of the Mouse Dihydrofolate Reductase Complementary Deoxyribonucleic Acid in Simian Virus 40", Molecular and Cellular Biology 2:854-864 (Sept. 1981) is digested with BamHI and EcoRI to yield the fragment 38 (5.0 kb) containing the ampicillin resistance gene, the SV40 origin, and the dhfr gene. The second portion of pSVCOW7 is obtained from plasmid pλGH2R2 which is digested with the same restriction endonucleases used to cleave pSV2dhfr to obtain fragment 39 containing the 3' end of genomic bovine growth hormone gene, i.e., BGH gDNA. Plasmid pλGH2R2 is publicly available from an E. coli HB101 host, deposited with the Northern Regional Research Laboratories in Peoria, Illinois (NRRL B-15154). Fragments 38 and 39 are ligated to yield pSVCOW7 (7.1 kb).

(2) Construction of pTPA-cDNA, BamHI - Chart 21.

In order to conveniently insert a TPA analog into pSVCOW7 it is necessary to insert a convenient BamHI site within the leader sequence of TPA which is upstream from the mature protein sequence. To accomplish this pTPA5'cDNA (Chart 5) is cut with HgaI and fragment 40 (517 bp) containing bases 78 to 593 is made flush with Klenow enzyme. The flush ends are ligated to a 10-mer BamHI linker and fragment 40 is cut with NarI to yield fragment 41 having bases 68 to 521 of the TPA cDNA.

Plasmid pTPA-cDNA (Chart 5) is cut with NarI and BgIII and fragment 42 (1644 bp) containing the 3' portion of the TPA cDNA is gel isolated. Fragments 41 and 42 are ligated to fragment 43 (4.3 kb) resulting from a BamHI and BgIII digestion of pKC7 to yield pTPA-cDNA, BamHI (6.5 kb).

(3) Construction of pTPA-IE-PA - Chart 22.

Plasmid pTPA-IE-PA containing a TPA modified cDNA having the native arrangement of TPA domains is capable of being expressed by CHO cells or capable of being used to facilitate the construction of alternative expression plasmid containing TPA analogs. The assembly of pTPA-IE-PA is accomplished in two steps. First the TPA cDNA from pTPA-cDNA is inserted into pSVCOW7 and then the immediate early promoter of cytomegalovirus is inserted to initiate transcription of the TPA analog.

STEP 1. Plasmid pSVCOW7 is cut with EcoRI and PuvII and fragment 44 (600 bp) containing the polyadenylation sequence of bovine growth hormone extending from the PvuII site in the 3' most exon of the BGH gene, to the EcoRI site downstream from the 3' end. For a complete discussion of the BGH polyadenylation sequence see the following references: (1) European patent application 0112012, published on 27 June 1984 wherein the identification and characterization of bGH genomic DNA is disclosed; (2) Woychik, R.P. et al., "Requirement for the 3' Flanking Region of the Bovine Growth Hormone Gene for Accurate Polyadenylation", Proc. Natl. Acad. Sci. USA 81:3944-3948 (July 1984); and, D.R. Higgs, et al., Nature 306:398-400 (24 November 1983) and references cited therein.

A second sample of pSVCOW7 is cut with EcoRI and BamHI to yield fragment 45. Fragment 45 can be alternatively derived from the EcoRI/BamHI fragment from parent plasmid pSV2dhfr available from Bethesda Research Laboratories. Fragment 45 contains the origin of replication from pBR322 and an ampicillin resistance gene expressed in E. coli which allows for the selection of the plasmid in E. coli. The fragment also contains the mouse dihydrofolate reductase cDNA in a construction that allows expression in mammalian cells. Subramani, et al., Mol. Cell. Biol. 1:854-864 (1981).

The TPA cDNA is obtained from fragment 46 (1.9 kb) which is obtained from cutting pTPA-cDNA,BamHI with BamHI and BalI. Fragment 46 contains the full coding region from tPA cDNA. The BamHI cut is in cDNA coding for the 5' untranslated sequences of the mRNA, and the BalI cut is in cDNA coding for the 3' untranslated region of the cDNA.

Fragments 44, 45 and 46 are ligated to form pTPA-PA (8.4 kb) which is a replication vector capable of shuttling between E coli and CHO cells. Plasmid pTPA-PA is transformed into E coli.

STEP 2. In step 2, pTPA-PA is converted into expression plasmid pTPA-IE-PA by inserting the immediate early gene promoter from human cytomegalovirus (CMV I.E. promoter). The CMV I.E. promoter is obtained from the PstI digestion of the CMV genome. The restriction endonuclease cleavage maps of the region of the human cytomegalovirus genome containing the major immediate early gene (CMV I.E.) have been described in detail (Stinski, et al., J. Virol. 46:1-14, 1983; Stenberg, et al., J. Virol. 49:190-199, 1984; and, Thomsen, et al., Proc. Natl. Acad. Sci. USA, 81:659-663, 1984).

These references describe a 2.0 kilobase PstI fragment which contains amongst its sequences the promoter for the major immediate early gene. The CMV I.E. promoter can be further isolated by isolation digestion of this 2.0 kb PstI fragment with Sau3AI, whereby a desired 760 base pair fragment is obtained among the products. This 760 basepair fragment can be distinguished from the other products by its size and the presence of a SacI cleavage site and a BalI cleavage site within the fragment. Because of its convenient identification, utilization of this Sau3AI fragment is the preferred method of using the CMV I.E. promoter as described in the present specification.

Plasmid pTPA-PA, constructed in Step 1, is cleaved with BamHI, and a Sau3AI fragment containing the CMV immediate early promoter is ligated into the BamHI site. Plasmids containing the CMV promoter fragment in an orientation such that transcription from the promoter would synthesize an mRNA for TPA are identified by cleavage of the plasmids with SacI. The resulting plasmid is designated pTPA-IE-PA having the CMV I.E. promoter at the 5'-end of the TPA cDNA and the bGH polyadenylation signal on its 3'-end.

(4) Construction of pTPA-IE-FK2K2A - Chart 23.

The construction of pTPA-IE-FK2K2A is a two step process. Step one involves the creation of pTPA-FK2K2A which contains the desired TPA analog, the polyadenylation signal sequence from BGH and the selectable markers and replicons of pSVCOW7 and Step 2 involves the insertion of the CMV I.E. promoter discussed earlier. While the example below describes the insertion of TPA analog FK2K2A, other analogs can be inserted using the same enzymes and procedures.

Step 1. Plasmid pTPA-IE-PA (Chart 22) is cut with BamHI and BglII to yield fragment 47 containing the TPA leader sequence bases 1-188. The polyadenylation signal sequence of BGH is obtained by cutting pSVCOW7 (Chart 20) with EcoRI and PvuII to yield fragment 48 (600bp). The TPA analog sequence is obtained from cutting pFK2K2A (chart 12) with BglII and BalI to obtain fragment 49 (2.0 kb). A second sample of pSVCOW7 is cut with EcoRI and BamHI to yield fragment 50 (5.8 kb) containing the markers and replicons of pSVCOW7. The four fragments are gel isolated and ligated using T4 ligase to yield pTPA-FK2K2A (8.3 kb).

Step 2. Plasmid pTPA-FK2K2A is cut with BamHI and the Sau3A digested CMV-IE promoter sequence is inserted to form pTPA-IE-FK2K2A which is maintained in E. coli until transfection into CHO cells. Transfection and Culturing of CHO Cells.

(5) Transfection and Culturing of CHO Cells.

Plasmid pTPA-IE-FK2K2A is transfected into Chinese hamster ovary (CHO) cells deficient in dihydrofolate reductase(dhfr) using the calcium phosphate method for transfection of DNA into cells which is described in detail by Graham, et al., (in Introduction of Macromolecules into Viable Mammalian Cells, Alan R. Liss Inc., N.Y. 1980, pp. 3-25). The cell line used is the mutant DXB-11 originally available from L Chasin, of Columbia University and completely described in Proc. Natl. Acad. Sci. USA 77:4216-4220, 1980). The above methods for transfection relies on the fact that cells which incorporate the transfected plasmids are not longer dhfr deficient and will grow in Dulbecco's modified Eagle's medium plus proline.

From the cells transfected with pTPA-IE-FK2K2A, clones are isolated, which, when grown in a monolayer for two days, synthesize at least 10 ng TPA per million cells. From cells with pIETPA-IPA-dhfr, clones are isolated which synthesize at least 100 ng TPA per million cells. Expression of TPA analog can be detected by radioimmunoassay, or Western blot techniques.

TPA analogs are purified according to the procedure of Rijken and Collen. Journal of Biological Chemistry 256:7035-7041 (1979). The CHO cells containing the recombinant TPA analogs are grown in serum free media. The culture media is harvested after 72 hours and applied to a zinc-chelate agarose column equilibrated with 0.02M Tris-HCl pH 7.5 containing 1.0M NaCl and 0.01% Tween 80. The column is washed with the same buffer and the TPA analogs eluted with a linear gradient from 0 to 0.05M imidazole in the same buffer. The fractions containing TPA analogs are pooled and applied to a concanavalin A-agarose column equilibrated with 0.01M phosphate buffer pH 7.5 containing 1.0M NaCl and 0.01% Tween 80. After washing the column with the same buffer, the TPA analogs are eluted with a linear gradient of the equilibration buffer to 0.01M phosphate buffer pH 7.5 containing 0.01% Tween® 80, 0.4M D-methylmannoside and 2M potassium thiocyanate. The fractions containing TPA activity are pooled and solid KSCN is added to increase the KSCN concentration to 1.6M. The fractions are concentrated approximately 10-fold and applied to a Sephadex G-150 column equilibrated with 0.01M phosphate buffer pH 7.5 containing 1.6M KSCN and 0.01% Tween® 80. The fractions containing the TPA activity are pooled, dialyzed against 0.15M NaCl and 0.01% Tween® 80 and stored at -80°C.

C. Expression in Spodoptera frugiperda.

The following example relates to the expression of TPA in insect cell cultures. All procedures are detailed in Summers, M.D. and Smith, G.E., A Manual for Baculovirus Vectors and Insect Cell Culture Procedures published by the College of Agriculture, Texas Agricultural Experiment Station, Texas Agricultural Extension Service, College Station, Texas, 1986. The starting plasmid pAc373 (7.1 kb) is a general baculovirus expression vector having a unique BamHI site immediately downstream from the polyhedron promoter for Autographa californica nuclear polyhedrosis virus (AcNPV). The polyhedron protein is a matrix protein that is nonessential for viral infection and replication in vitro. The plasmid is available from Professor Max Summers of the Department of Entomology, Texas A&M University, College Station, Texas 77843 and is fully described in Molecular and Cell. Biology, 3(12):2156-2165 (1983).

(1) Construction of pAcTPA - Chart 24.

Starting plasmid pAc373 is modified to accept the TPA analogs by inserting into the unique BamHI site a BglII site which is also unique. Plasmid pAc373 is first cut with BamHI and then treated with Mung bean nuclease to create blunt ends. BglII linkers are then ligated to the ends and the ends rejoined to yield pAc373,BglII. Plasmid pTPAcDNA,BamHI from chart 21 is fully digested with BamHI and partially digested with BglII to yield fragment 51 (1.95 Kb) coding for an intact TPA cDNA. Fragment 51 is gel isolated and inserted into the BglII site of pAc373,BglII to yield pAcTPA which is capable of expressing native TPA in S. frugiperda.

(2) Construction of pAcFK2K2A - Chart 25.

For this expression plasmid the TPA analog FK2K2A is cut from pFK2K2A (Chart 12) using BglII and the cDNA encoding the TPA analog is gel isolated as fragment 53 (2.0 kb). Plasmid pAcTPA (Chart 24) is cut with BglII to yield fragment 52 (7.15 kb). Fragments 52 and 53 are ligated to yield pAcFK2K2A (9.15 kb) which is maintained in E. coli until transfected into S. frugiperda.

(3) Transfection and culturing of S. frugiperda.

The TPA analogs are recombined with native ACNPV DNA by cotransfection in S. frugiperda. S. frugiperda (SF9; ATCC CRL 1711) are cultured in Grace Media (Gibco Lab. Livonia, MI 48150), 10% fetal calf serum and supplemented with Difco Lactalbumin hydrolysate and yeastolate. The cells are cotransfected with AcNPV DNA and pAcTPAFK2K2A at $1\mu$/ml and $2\mu$/ml respectively. Resulting virus particles are obtained by collecting the media and removing cellular material by low spread centrifugation. The virus containing-media is then used to infect S. frugiperda. Subsequent infection of S. frugiperda using these viral particles which include both native viral DNA and DNA recombined with the cDNA coding for the FK2K2A TPA analog will result in some cells expressing the TPA analog instead of the polyhedron protein. Detection of infected cell colonies producing TPA analogs is determined by overlaying a monolayer of S. frugiperda that has been previously infected for one hour with serial dilutions of the viral containing media ($10^{-1}$-$10^{-6}$). The cells are then overlayed with Grace media with 0.7% low melting point agar containing 6 mg/ml fibrinogen and 0.5 units of thrombin. Those cells producing active TPA will form clear plaques around the foci of infection.

Example 5.

FK2A has been isolated from CHO cells and evaluated for activity and antigenicity. Relative to a standard TPA preparation, the respective activities of purified native recombinant DNA and of FK2A are 49,000 and 4.2 ± 0.06 l/ml. Using the goat polyclonal antibody to TPA described above, the respective antigen measurements were 912,000 and 97 ± 28 ng/ml.

FK2A forms complexes with a plasminogen activator inhibitor from platelets (thrombin-induced platelet releasate). The molecular weight of FK2A is about 40,000 daltons. The molecular weight of the enzyme inhibitor complex is approximately 85,000 daltons.

TPA is known to be therapeutically useful for dissolving thrombi; see The Lancet, pp. 1018-20 (Nov. 7, 1981); Science 220:1181 (1983); and N. Eng. J. Med. 310:609 (1984). Administration of TPA analogues to patients with coronary thrombi can be via intracoronary or intravenous routes according to the general procedures described in these references.

## TABLE I

```
   1  TTCTGAGCACAGGGCTGGAGAGAAAACCTCTGCGAGGAAAGGGAAGGAGCAAGCCGTGAA
      ||||||||||||||||||||||||||||||||||||||||||||||||||||||||||||
   1  TTCTGAGCACAGGGCTGGAGAGAAAACCTCTGCGAGGAAAGGGAAGGAGCAAGCCGTGAA

  61  TTTAAGGGACGCTGTGAAGCAATCATGGATGCAATGAAGAGAGGGCTCTGCTGTGTGCTG
      ||||||||||||||||||||||||||||||||||||||||||||||||||||||||||||
  61  TTTAAGGGACGCTGTGAAGCAATCATGGATGCAATGAAGAGAGGGCTCTGCTGTGTGCTG

 121  CTGCTGTGTGGAGCAGTCTTCGTTTCGCCCAGCCAGGAAATCCATGCCCGATTCAGAAGA
      ||||||||||||||||||||||||||||||||||||||||||||||||||||||||||||
 121  CTGCTGTGTGGAGCAGTCTTCGTTTCGCCCAGCCAGGAAATCCATGCCCGATTCAGAAGA

 181  GGAGCCAGATCT    TACCAAGTGATCTGCAGAGATGAAAAAACGCAGATGATATACCAG
      |||||||||||    ||||||||||||||||||||||||||||||||||||||||||||||
 181  GGAGCCAGATCTAGATACCAAGTGATCTGCAGAGATGAAAAAACGCAGATGATATACCAG

 238  CAACATCAGTCATGGCTGCGCCCTGTGCTCAGAAGCAACCGGGTGGAATATTGCTGGTGC
      ||||||||||||||||||||||||||||||||||||||||||||||||||||||||||||
 241  CAACATCAGTCATGGCTGCGCCCTGTGCTCAGAAGCAACCGGGTGGAATATTGCTGGTGC

 298  AACAGTGGCAGGGCACAGTGCCACTCAGTGCCTGTCAAAAGTTGCAGCGAGCCAAGGTGT
      |||||||||||||||||||||||||||||||||||| ||    |||||||||||||||||
 301  AACAGTGGCAGGGCACAGTGCCACTCAGTGCCTGTTAACGCATGCAGCGAGCCAAGGTGT

 358  TTCAACGGGGGCACCTGCCAGCAGGCCCTGTACTTCTCAGATTTCGTGTGCCAGTGCCCC
      ||||||||||||||||||||||||||||||||||||||||||||||||||||||||||||
 361  TTCAACGGGGGCACCTGCCAGCAGGCCCTGTACTTCTCAGATTTCGTGTGCCAGTGCCCC

 418  GAAGGATTTGCTGGGAAGTGCTGTGAAATAGATACCAGGGCCACGTGCTACGAGGACCAG-
      |||||||||||||||||||||||||| || |||||| |||||||||||||||||||||||
 421  GAAGGATTTGCTGGGAAGTGCTGTGATATCGATACCCGGGCCACGTGCTACGAGGACCAG

 478  GGCATCAGCTACAGGGGCACGTGGAGCACAGCGGAGAGTGGCGCCGAGTGCACCAACTGG
      ||||||||||||||||||||||||||||||||||||||||||||||||||||||||||||
 481  GGCATCAGCTACAGGGGCACGTGGAGCACAGCGGAGAGTGGCGCCGAGTGCACCAACTGG

 538  AACAGCAGCGCGTTGGCCCCAGAAGCCCTACAGCGGGCCGGAGGCCAGACGCCATCAGGCTG
      |||||||||||||||||||||||||||||||||||||||||||||||||||||||||||
 541  AACAGCAGCGCGTTGGCCCCAGAAGCCCTACAGCGGGCCGGAGGCCAGACGCCATCAGGCTG

 598  GGCCTGGGGAACCACAACTACTGCAGAAACCCAGATCGAGACTCAAAGCCCTGGTGCTAC
      ||||||||||||||||||||||||||||||||||||||||||||||||||||||||||||
 601  GGCCTGGGGAACCACAACTACTGCAGAAACCCAGATCGAGACTCAAAGCCCTGGTGCTAC

 658  GTCTTTAAGGCGGGGAAGTACAGCTCAGAGTTCTGCAGCACCCCTGCCTGCTCTGAGCGA
      ||||||||||||||||||||||||||||||||||||||||||||||||||||||||||||
 661  GTCTTTAAGGCGGGGAAGTACAGCTCAGAGTTCTGCAGCACCCCTGCCTGCTCTGAGCGA

 718  AAC    AGTGACTGCTACTTTGGGAATGGGTCAGCCTACCGTGGCACGCACAGCCTCACC
      | |    ||||||||||||||||||||||||||||||||||||||||||||||||||||||
 721  TCCGTTAACGACTGCTACTTTGGGAATGGGTCAGCCTACCGTGGCACGCACAGCCTCACC
```

23

```
 775   CAGTCGGGTGCCTCCTGCCTCCCGTGGAATTCCATGATCCTGATAGGCAAGGTTTACACA
       ||||||||||||||||||||||||||||||||||||||||||||||||||||||||||||
 781   GAGTCGGGTGCCTCCTGCCTCCCGTGGAATTCCATGATCCTGATAGGCAAGGTTTACACA

 835   GCACAGAACCCCAGTGCCCAGGCACTGGGCCTGGGCAAACATAATTACTGCCGGAATCCT
       ||||||||||||||||||||||||||||||||||||||||||||||||||||||||||||
 841   GCACAGAACCCCAGTGCCCAGGCACTGGGCCTGGGCAAACATAATTACTGCCGGAATCCT

 895   GATGGGGATGCCAAGCCCTGGTGCCACGTGCTGAAGAACCGCAGGCTGACGTGGGAGTAC
       ||||||||||||||||||||||||||||||||||||||||||||||||||||||||||||
 901   GATGGGGATGCCAAGCCCTGGTGCCACGTGCTGAAGAACCGCAGGCTGACGTGGGAGTAC

 955   TGTGATGTGCCCTCCTGCTCCACC   TGCGGCCTGAGACAGTACAGCCAGCCTCAGTTT
       |||||||||||||||||| | |||   ||||||| ||||   ||||||||||||||||||
 961   TGTGATGTGCCCTCCTGCGCAACCGCATGCGGCCGGAGA   TACAGCCAGCCTCAGTTT

1012   CGCATCAAAGGAGGGCTCTTCGCCGACATCGCCTCCCACCCCTGGCAGGCTGCCATCTTT
       ||||||||||||||||||||||||||||||||||||||||||||||||||||||||||||
1021   CGCATCAAAGGAGGGCTCTTCGCCGACATCGCCTCCCACCCCTGGCAGGCTGCCATCTTT

1072   GCCAAGCACAGGAGGTCGCCCGGAGAGCGGTTCCTGTGCGGGGGCATACTCATCAGCTCC
       ||||||||||||||||||||||||||||||||||||||||||||||||||||||||||||
1081   GCCAAGCACAGGAGGTCGCCCGGAGAGCGGTTCCTGTGCGGGGGCATACTCATCAGCTCC

1132   TGCTGGATTCTCTCTGCCGCCCACTGCTTCCAGGAGAGGTTTCCGCCCCACCACCTGACG
       ||||||||||||||||||||||||||||||||||||||||||||||||||||||||||||
1141   TGCTGGATTCTCTCTGCCGCCCACTGCTTCCAGGAGAGGTTTCCGCCCCACCACCTGACG

1192   GTGATCTTGGGCAGAACATACCGGGTGGTCCCTGGCGAGGAGGAGCAGAAATTTGAAGTC
       ||||||||||||||||||||||||||||||||||||||||||||||||||||||||||||
1201   GTGATCTTGGGCAGAACATACCGGGTGGTCCCTGGCGAGGAGGAGCAGAAATTTGAAGTC

1252   GAAAAATACATTGTCCATAAGGAATTCGATGATGACACTTACGACAATGACATTGCGCTG
       ||||||||||||||||||||||||||||||||||||||||||||||||||||||||||||
1261   GAAAAATACATTGTCCATAAGGAATTCGATGATGACACTTACGACAATGACATTGCGCTG

1312   CTGCAGCTGAAATCGGATTCGTCCCGCTGTGCCCAGGAGAGCAGCGTGGTCCGCACTGTG
       ||||||||||||||||||||||||||||||||||||||||||||||||||||||||||||
1321   CTGCAGCTGAAATCGGATTCGTCCCGCTGTGCCCAGGAGAGCAGCGTGGTCCGCACTGTG

1372   TGCCTTCCCCCGGCGGACCTGCAGCTGCCGGACTGGACGGAGTGTGAGCTCTCCGGCTAC
       ||||||||||||||||||||||||||||||||||||||||||||||||||||||||||||
1381   TGCCTTCCCCCGGCGGACCTGCAGCTGCCGGACTGGACGGAGTGTGAGCTCTCCGGCTAC

1432   GGCAAGCATGAGGCCTTGTCTCCTTTCTATTCGGAGCGGCTGAAGGAGGCTCATGTCAGA
       ||||||||||||||||||||||||||||||||||||||||||||||||||||||||||||
1441   GGCAAGCATGAGGCCTTGTCTCCTTTCTATTCGGAGCGGCTGAAGGAGGCTCATGTCAGA

1492   CTGTACCCATCCAGCCGCTGCACATCACAACATTTACTTAACAGAACAGTCACCGACAAC
       ||||||||||||||||||||||||||||||||||||||||||||||||||||||||||||
1501   CTGTACCCATCCAGCCGCTGCACATCACAACATTTACTTAACAGAACAGTCACCGACAAC
```

```
1552  ATGCTGTGTGCTGGAGACACTCGGAGCGGCGGGCCCCAGGCAAACTTGCACGACGCCTGC
      ||||||||||||||||||||||||||||||||||||||||||||||||||||||||||||
1561  ATGCTGTGTGCTGGAGACACTCGGAGCGGCGGGCCCCAGGCAAACTTGCACGACGCCTGC

1612  CAGGGCGATTCGGGAGGCCCCCTGGTGTGTCTGAACGATGGCCGCATGACTTTGGTGGGC
      ||||||||||||||||||||||||||||||||||||||||||||||||||||||||||||
1621  CAGGGCGATTCGGGAGGCCCCCTGGTGTGTCTGAACGATGGCCGCATGACTTTGGTGGGC

1672  ATCATCAGCTGGGGCCTGGGCTGTGGACAGAAGGATGTCCCGGGTGTGTACACAAAGGTT
      ||||||||||||||||||||||||||||||||||||||||||||||||||||||||||||
1681  ATCATCAGCTGGGGCCTGGGCTGTGGACAGAAGGATGTCCCGGGTGTGTACACAAAGGTT

1732  ACCAACTACCTAGACTGGATTCGTGACAACATGCGACCGTGACCAGGAACACCCGACTCC
      ||||||||||||||||||||||||||||||||||||||||||||||||||||||||||||
1741  ACCAACTACCTAGACTGGATTCGTGACAACATGCGACCGTGACCAGGAACACCCGACTCC

1792  TCAAAAGCAAATGAGATCCCGCCTCTTCTTCTTCAGAAGACACTGCAAAGGCGCAGTGCT
      ||||||||||||||||||||||||||||||||||||||||||||||||||||||||||||
1801  TCAAAAGCAAATGAGATCCCGCCTCTTCTTCTTCAGAAGACACTGCAAAGGCGCAGTGCT

1852  TCTCTACAGACTTCTCCAGACCCACCACACCGCAGAAGCGGGACGAGACCCTACAGGAGA
      ||||||||||||||||||||||||||||||||||||||||||||||||||||||||||||
1861  TCTCTACAGACTTCTCCAGACCCACCACACCGCAGAAGCGGGACGAGACCCTACAGGAGA

1912  GGGAAGAGTGCATTTTCCCAGATACTTCCCATTTTGGAAGTTTTCAGGACTTGGTCTGAT
      ||||||||||||||||||||||||||||||||||||||||||||||||||||||||||||
1921  GGGAAGAGTGCATTTTCCCAGATACTTCCCATTTTGGAAGTTTTCAGGACTTGGTCTGAT

1972  TTCAGGATACTCTGTCAGATGGGAAGACATGAATGCACACTAGCCTCTCCAGGAATGCCT
      ||||||||||||||||||||||||||||||||||||||||||||||||||||||||||||
1981  TTCAGGATACTCTGTCAGATGGGAAGACATGAATGCACACTAGCCTCTCCAGGAATGCCT

2032  CCTCCCTGGGCAGAAGTGGCCATGCCACCCTGTTTTCGCTAAAGCCCAACCTCCTGACCT
      ||||||||||||||||||||||||||||||||||||||||||||||||||||||||||||
2041  CCTCCCTGGGCAGAAGTGGCCATGCCACCCTGTTTTCGCTAAAGCCCAACCTCCTGACCT

2092  GTCACCGTGAGCAGCTTTGGAAACAGGACCACAAAAAATGAAAGCATGTCTCAATAGTAAA
      ||||||||||||||||||||||||||||||||||||||||||||||||||||||||||||
2101  GTCACCGTGAGCAGCTTTGGAAACAGGACCACAAAAAATGAAAGCATGTCTCAATAGTAAA

2152  AGAAACAAGAGATCTTTCAGGAAAGACGGATTGCATTAGAAATAGACAGTATATTTATAG
      ||||||||||||||||||||||||||||||||||||||||||||||||||||||||||||
2161  AGAAACAAGAGATCTTTCAGGAAAGACGGATTGCATTAGAAATAGACAGTATATTTATAG

2212  TCACAAGGGCCCAGCAGGGCTCAAAGTTGGGGCAGGCTGGCTGGCCCGTCATGTTCCTCA
      ||||||||||||||||||||||||||||||||||||||||||||||||||||||||||||
2221  TCACAAGGGCCCAGCAGGGCTCAAAGTTGGGGCAGGCTGGCTGGCCCGTCATGTTCCTCA

2272  AAAGCGCCCTTGACGTCAAGTCTCCTTCCCCTTTCCCCACTCCCTGGCTCTCAGAACGTA
      ||||||||||||||||||||||||||||||||||||||||||||||||||||||||||||
2821  AAAGCGCCCTTGACGTCAAGTCTCCTTCCCCTTTCCCCACTCCCTGGCTCTCAGAACGTA
```

```
2332   TTCCTTTTGAGTACAGTGTGTAAAGTGTAAATCCTTTTTCTTTATAAACTTTAGAGTAGC
       ||||||||||||||||||||||||||||||||||||||||||||||||||||||||||||
2341   TTCCTTTTGAGTACAGTGTGTAAAGTGTAAATCCTTTTTCTTTATAAACTTTAGAGTAGC

2392   ATGAGAGAATTGTATCATTTGAACAACTAGGCTTCAGCATATTTATAGCGATCCATCGTT
       ||||||||||||||||||||||||||||||||||||||||||||||||||||||||||||
2401   ATGAGAGAATTGTATCATTTGAACAACTAGGCTTCAGCATATTTATAGCGATCCATCGTT

2452   AGTTTTTACTTTCCGTTGCCACAACCCTGTTTTATACCGTACTTAATAAATTCGGATATA
       ||||||||||||||||||||||||||||||||||||||||||||||||||||||||||||
2461   AGTTTTTACTTTCCGTTGCCACAACCCTGTTTTATACCGTACTTAATAAATTCGGATATA

2512   TTTTTTCACAGTTTTTTCCAAAAAAAAAAAAAAAA
       |||||||||||||||||||||||||||||||||||
2521   TTTTTTCACAGTTTTTTCCAAAAAAAAAAAAAAAA
```

Matches - 2526    Mismatches - 15       Unmatched - 12
Length - 2553      Matches/length - 99.0 percent

## TABLE 2

```
  1   MetAspAlaMetLysArgGlyLeuCysCysValLeuLeuLeuCysGlyAlaValPheVal
       |  |  |  |  |  |  |  |  |  |  |  |  |  |  |  |  |  |  |  |
      MetAspAlaMetLysArgGlyLeuCysCysValLeuLeuLeuCysGlyAlaValPheVal

 21   SerProSerGlnGluIleHisAlaArgPheArgArgGlyAlaArgSer   TyrGlnVal
       |  |  |  |  |  |  |  |  |  |  |  |  |  |  |  |     |  |  |
 21   SerProSerGlnGluIleHisAlaArgPheArgArgGlyAlaArgSerArgTyrGlnVal

 40   IleCysArgAspGluLysThrGlnMetIleTyrGlnGlnHisGlnSerTrpLeuArgPro
       |  |  |  |  |  |  |  |  |  |  |  |  |  |  |  |  |  |  |  |
 41   IleCysArgAspGluLysThrGlnMetIleTyrGlnGlnHisGlnSerTrpLeuArgPro

 60   ValLeuArgSerAsnArgValGluTyrCysTrpCysAsnSerGlyArgAlaGlnCysHis
       |  |  |  |  |  |  |  |  |  |  |  |  |  |  |  |  |  |  |  |
 61   ValLeuArgSerAsnArgValGluTyrCysTrpCysAsnSerGlyArgAlaGlnCysHis

 80   SerValProValLysSerCysSerGluProArgCysPheAsnGlyGlyThrCysGlnGln
       |  |  |  |  |  |  |  |  |  |  |  |  |  |  |  |  |  |  |  |
 81   SerValProValAsnAlaCysSerGluProArgCysPheAsnGlyGlyThrCysGlnGln

100   AlaLeuTyrPheSerAspPheValCysGlnCysProGluGlyPheAlaGlyLysCysCys
       |  |  |  |  |  |  |  |  |  |  |  |  |  |  |  |  |  |  |  |
101   AlaLeuTyrPheSerAspPheValCysGlnCysProGluGlyPheAlaGlyLysCysCys

120   GluIleAspThrArgAlaThrCysTyrGluAspGlnGlyIleSerTyrArgGlyThrTrp
       |  |  |  |  |  |  |  |  |  |  |  |  |  |  |  |  |  |  |  |
121   AspIleAspThrArgAlaThrCysTyrGluAspGlnGlyIleSerTyrArgGlyThrTrp

140   SerThrAlaGluSerGlyAlaGluCysThrAsnTrpAsnSerSerAlaLeuAlaGlnLys
       |  |  |  |  |  |  |  |  |  |  |  |  |  |  |  |  |  |  |  |
141   SerThrAlaGluSerGlyAlaGluCysThrAsnTrpAsnSerSerAlaLeuAlaGlnLys

160   ProTyrSerGlyArgArgProAspAlaIleArgLeuGlyLeuGlyAsnHisAsnTyrCys
       |  |  |  |  |  |  |  |  |  |  |  |  |  |  |  |  |  |  |  |
161   ProTyrSerGlyArgArgProAspAlaIleArgLeuGlyLeuGlyAsnHisAsnTyrCys

180   ArgAsnProAspArgAspSerLysProTrpCysTyrValPheLysAlaGlyLysTyrSer
       |  |  |  |  |  |  |  |  |  |  |  |  |  |  |  |  |  |  |  |
181   ArgAsnProAspArgAspSerLysProTrpCysTyrValPheLysAlaGlyLysTyrSer

200   SerGluPheCysSerThrProAlaCysSerGluGly   AsnSerAspCysTyrPheGly
       |  |  |  |  |  |  |  |  |  |  |  |     |  |  |  |  |  |
201   SerGluPheCysSerThrProAlaCysSerGluGlySerValAsnAspCysTyrPheGly

219   AsnGlySerAlaTyrArgGlyThrHisSerLeuThrGluSerGlyAlaSerCysLeuPro
       |  |  |  |  |  |  |  |  |  |  |  |  |  |  |  |  |  |  |  |
221   AsnGlySerAlaTyrArgGlyThrHisSerLeuThrGluSerGlyAlaSerCysLeuPro

239   TrpAsnSerMetIleLeuIleGlyLysValTyrThrAlaGlnAsnProSerAlaGlnAla
       |  |  |  |  |  |  |  |  |  |  |  |  |  |  |  |  |  |  |  |
241   TrpAsnSerMetIleLeuIleGlyLysValTyrThrAlaGlnAsnProSerAlaGlnAla
```

27

```
259  LeuGlyLeuGlyLysHisAsnTyrCysArgAsnProAspGlyAspAlaLysProTrpCys
     | | | | | | | | | | | | | | | | | | | |
261  LeuGlyLeuGlyLysHisAsnTyrCysArgAsnProAspGlyAspAlaLysProTrpCys

279  HisValLeuLysAsnArgArgLeuThrTrpGluTyrCysAspValProSerCysSerThr
     | | | | | | | | | | | | | | | | | | | |
281  HisValLeuLysAsnArgArgLeuThrTrpGluTyrCysAspValProSerCysAlaThr

299      CysGlyLeuArgGlnTyrSerGlnProGlnPheArgIleLysGlyGlyLeuPheAla
         | | | |      | | | | | | | | | | | | | |
301  AlaCysGlyArgArg    TyrSerGlnProGlnPheArgIleLysGlyGlyLeuPheAla

318  AspIleAlaSerHisProTrpGlnAlaAlaIlePheAlaLysHisArgArgSerProGly
     | | | | | | | | | | | | | | | | | | | |
321  AspIleAlaSerHisProTrpGlnAlaAlaIlePheAlaLysHisArgArgSerProGly

338  GluArgPheLeuCysGlyGlyIleLeuIleSerSerCysTrpIleLeuSerAlaAlaHis
     | | | | | | | | | | | | | | | | | | | |
341  GluArgPheLeuCysGlyGlyIleLeuIleSerSerCysTrpIleLeuSerAlaAlaHis

358  CysPheGlnGluArgPheProProHisHisLeuThrValIleLeuGlyArgThrTyrArg
     | | | | | | | | | | | | | | | | | | | |
361  CysPheGlnGluArgPheProProHisHisLeuThrValIleLeuGlyArgThrTyrArg

378  ValValProGlyGluGluGluGlnLysPheGluValGluLysTyrIleValHisLysGlu
     | | | | | | | | | | | | | | | | | | | |
381  ValValProGlyGluGluGluGlnLysPheGluValGluLysTyrIleValHisLysGlu

398  PheAspAspAspThrTyrAspAsnAspIleAlaLeuLeuGlnLeuLysSerAspSerSer
     | | | | | | | | | | | | | | | | | | | |
401  PheAspAspAspThrTyrAspAsnAspIleAlaLeuLeuGlnLeuLysSerAspSerSer

418  ArgCysAlaGlnGluSerSerValValArgThrValCysLeuProProAlaAspLeuGln
     | | | | | | | | | | | | | | | | | | | |
421  ArgCysAlaGlnGluSerSerValValArgThrValCysLeuProProAlaAspLeuGln

438  LeuProAspTrpThrGluCysGluLeuSerGlyTyrGlyLysHisGluAlaLeuSerPro
     | | | | | | | | | | | | | | | | | | | |
441  LeuProAspTrpThrGluCysGluLeuSerGlyTyrGlyLysHisGluAlaLeuSerPro

458  PheTyrSerGluArgLeuLysGluAlaHisValArgLeuTyrProSerSerArgCysThr
     | | | | | | | | | | | | | | | | | | | |
461  PheTyrSerGluArgLeuLysGluAlaHisValArgLeuTyrProSerSerArgCysThr

478  SerGlnHisLeuLeuAsnArgThrValThrAspAsnMetLeuCysAlaGlyAspThrArg
     | | | | | | | | | | | | | | | | | | | |
481  SerGlnHisLeuLeuAsnArgThrValThrAspAsnMetLeuCysAlaGlyAspThrArg

498  SerGlyGlyProGlnAlaAsnLeuHisAspAlaCysGlnGlyAspSerGlyGlyProLeu
     | | | | | | | | | | | | | | | | | | | |
501  SerGlyGlyProGlnAlaAsnLeuHisAspAlaCysGlnGlyAspSerGlyGlyProLeu
```

28

```
518    ValCysLeuAsnAspGlyArgMetThrLeuValGlyIleIleSerTrpGlyLeuGlyCys
       |  |  |  |  |  |  |  |  |  |  |  |  |  |  |  |  |  |  |  |
521    ValCysLeuAsnAspGlyArgMetThrLeuValGlyIleIleSerTrpGlyLeuGlyCys

538    GlyGlnLysAspValProGlyValTyrThrLysValThrAsnTyrLeuAspTrpIleArg
       |  |  |  |  |  |  |  |  |  |  |  |  |  |  |  |  |  |  |  |
541    GlyGlnLysAspValProGlyValTyrThrLysValThrAsnTyrLeuAspTrpIleArg

558    AspAsnMetArgProEnd
       |  |  |  |  |  |
561    AspAsnMetArgProEnd
```

Matches - 556     Mismatches - 7     Unmatched - 3
Length - 566      Matches/length - 98.2 percent

## TABLE 3

### TPA OLIGONUCLEOTIDES

BLOCK 1 - FINGER DOMAIN

P1  5'GATCTAGATACCAAGTG
P2  5'ATCTGCAGAGATGAAAAAACGC
P3  5'AGATGATATACCAGCAACAT
P4  5'CAGTCATGGCTGCGCCCTGTGC
P5  5'TCAGAAGCAACCGGGTGGAAT
P6  5'ATTGCTGGTGCAACAGTGGCAG
P7  5'AGCACAGTGTCACTCAGTTCCTGTTAACGCATG
P8  5'GCAGATCACTTGGTATCTA
P9  5'ATCATCTGCGTTTTTTTCATCTCT
P10 5'ATGACTGATGTTGCTGGTAT
P11 5'CTTCTGAGCACAGGGCGCAGCC
P12 5'AGCAATATTCCACCCGGTTG
P13 5'TGTGCTCTGCCACTGTTGCACC
P14 5'CGTTAACAGGAACTGAGTGACAC

BLOCK 2 - GROWTH FACTOR DOMAIN

P15 5'CAGCGAGCCAAGGTGTTTCAACGGGGGCACC
P16 5'TGCCAGCAGGCCCTGTACTTCTCAGATTTC
P17 5'GTGTGCCAGTGCCCCCGAAGG
P18 5'ATTTGCTGGGAAGTGCTGTGATAT
P19 5'GAAACACCTTGGCTCGCTGCATG
P20 5'CCTGCTGGCAGGTGCCCCCGTT
P21 5'GGCACACGAAATCTGAGAAGTACAGGG
P22 5'AGCAAATCCTTCGGGGCACT
P23 5'CGATATCACAGCACTTCCC

BLOCK 3 - KRINGLE 1 DOMAIN

P24 5'CGATACCCGGGCCACGTGCTAC
P25 5'GAGGACCAGGGCATCAGCTACAGG
P26 5'GGCACGTGGAGCACAGCGGAG
P27 5'AGTGGCGCCGAGTGCACCAACTGGAACAG
P28 5'CAGCGCGTTGGCCCAGAAGCCCTAC
P29 5'AGCGGGCGGAGGCCAGACGCCATCA
P30 5'GGCTGGGCCTGGGGAACCACAACTACTGCAG
P31 5'AAACCCAGATCGAGACTCAAAGCCCTGGTGC
P32 5'TACGTCTTTAAGGCGGGGAAGTACAGCTCAGAG
P33 5'TTCTGCAGCACCCCTGCCTGCTCTGAGG
P34 5'GTCCTCGTAGCACGTGGCCCGGGTAT
P35 5'CGTGCCCCTGTAGCTGATGCCCTG
P36 5'CGCCACTCTCCGCTGTGCTCCA
P37 5'ACGCGCTGCTGTTCCAGTTGGTGCACTCGG
P38 5'CCCGCTGTAGGGCTTCTGGGCCA
P39 5'CCAGCCTGATGGCGTCTGGCCTCCG
P40 5'CTGGGTTTCTGCAGTAGTTGTGGTTCCCCAGGC
P41 5'AAAGACGTAGCACCAGGGCTTTGAGTCTCGAT
P42 5'GCAGAACTCTGAGCTGTACTTCCCCGCCTT
P43 5'GATCCCTCAGAGCAGGCAGGGGTGCT

BLOCK 4 - KRINGLE 2 DOMAIN

P44 5'GATCCGTTAACGACTGCTACTT
P45 5'TGGGAATGGGTCAGCCTACC
P46 5'GTGGCACGCACAGCCTCACCGAG
P47 5'TCGGGTGCCTCCTGCCTCCCGTGGA
P48 5'ATTCCATGATCCTGATAGGCAAG
P49 5'GTTTACACAGCACAGAACCCCAGTGCGC
P50 5'AGGCACTGGGGCTCGGGAAAC
P51 5'ATAATTACTGCCGGAATCCTGATG
P52 5'GGGATGCCAAGCCCTGGTGCCACGT
P53 5'GCTGAAGAACCGCAGGCTGACGTGG
P57 5'AAAGGAGGGCTCTTCGCCGACATCGCCT
P58 5'CCCACCCCTGGCAGGCTGC
P59 5'CATCTTTGCCAAGCACAGGAGGTCGCC
P60 5'CGGAGAGCGGTTCCTGTGCG
P61 5'GGGGCATACTCATCAGCTCCTGCTGGAT
P62 5'TCTCTCTGCCGCCCACTGCTTCCA
P63 5'GGAGAGGTTTCCGCCCCACCAC
P64 5'CTGACGGTGATCTTGGGCAGAACATAC
P65 5'CGGGTGGTCCCTGGCGAGGAGGAGCAGAAAT
P66 5'TTGAAGTCGAAAAATACATTGTCCATAAGG
P67 5'TTCCCAAAGTAGCAGTCGTTAACG
P68 5'TGCCACGGTAGGCTGACCCA
P69 5'CACCCGACTCGGTGAGGCTGTGCG
P70 5'CATGGAATTCCACGGGAGGCAGGAGG
P71 5'GTAAACCTTGCCTATCAGGAT
P72 5'AGTGCCTGCGCACTGGGGTTCTGTGCTGT
P73 5'AGTAATTATGTTTCCCGAGCCCC
P74 5'CATCCCCATCAGGATTCCGGC
P75 5'CTTCAGCACGTGGCACCAGGGCTTGG
P76 5'AGTACTCCCACGTCAGCGTGCGGTT
P80 5'CCAGGGGTGGGAGGCGATGTCGGCGAAGAGCC
P81 5'TTGGCAAAGATGGCAGCCTG
P82 5'TCTCCGGGCGACCTCCTGTGC
P83 5'ATGCCCCCGCACAGGAACCGC
P84 5'AGAGAGAATCCAGCAGGAGCTGATGAGT
P85 5'CTCTCCTGGAAGCAGTGGGCGGC
P86 5'CGTCAGGTGGTGGGGCGGAAAC
P87 5'CACCCGGTATGTTCTGCCCAAGATCAC
P88 5'GACTTCAAATTTCTGCTCCTCCTCGCCAGGGAC
P89 5'AATTCCTTATGGACAATGTATTTTTC
P92 5'GAGTACTGTGATGTGCCCTCCTGCGCAACCGCATGC
P93 5'GGCCGGAGATACAGCCAGCCTCAGTTTCGCATC
P94 5'GGCCGCATGCGGTTGCGCAGGAGGGCACATCAC
P95 5'CTCCTTTGATGCGAAACTGAGGCTGGCTGTATCTCC

## TABLE 4

TPA DOMAINS

| Domains | Native TPA |
|---|---|
| Finger | Serine #1 → Lysine #49<br>Nucleotides 190 → 336 |
| Growth Factor | Serine #50 → Threonine #91<br>Nucleotides 337 → 462 |
| Kringle 1 | Cysteine #92 → Serine #174<br>Nucleotides 463 → 711 |
| Kringle 2 | Glutamic acid #175 → Serine #262<br>Nucleotides 712 → 975 |
| Active Site | Threonine #263 → Proline #527<br>Nucleotides 976 → 1770 |

TPA ANALOG (CHART 11)

| Domains | |
|---|---|
| Finger | Serine #1 → Valine #49<br>Nucleotides 190 → 342 |
| Growth Factor | Alanine #51 → Alanine #91<br>Nucleotides 343 → 456 |
| Kringle 1 | Threonine #92 → Cysteine #175<br>Nucleotides 457 → 726 |
| Kringle 2 | Glutamic acid #176 → Alanine #266<br>Nucleotides 727 → 999 |
| Active Site | Cysteine #267 → Proline #530<br>Nucleotides 1000 → 1782 |

CHART 1. THE CONSTRUCTION OF pTRZ1.

(a) pMC1403 is treated with EcoRI, Klenow enzyme and bacterial alkaline phosphatase to yield fragment 1.

**Fragment 1**

```
        SmaI   BamHI                    SalI                 EcoRI
   5'  |___|_____|_____|_____|__  3'
            |        |         |                    |
          LacZ     LacY      LacA                  AmpR
```

(b) pVV1 is treated with PvuII, BglII and Klenow enzyme followed by purification of fragment 2 (323 bp) containing the trp promoter and part of TrpLE.

**Fragment 2**

```
          PvuII              HpaI                      BglII
     5'  |_____|_____|__  3'
              |                      |
            Ptrp                   trpLE
```

(c) Fragments 1 and 2 are ligated using T4 DNA ligase to yield pTRZ1 (10 kb).

**pTRZ1**

```
        EcoRI             HpaI              SmaI  BamHI              SalI
   *5' |___|_____|_____|_____|_____|_____|__  3'*
           |            |            |      |      |      |     |
         Ptrp         trpLE        LacZ   LacY   LacA  AmpR
```

AmpR = Ampicillin resistance.
LacZ, LacY, LacA = Genes in the lactose operon.
Ptrp = Trp promoter/operator.
trpLE = Fusion of trpL and trpE.

CHART 2. CONSTRUCTION OF pSK3 (4.3 kb).

(a) pTRZ1 (10 kb) is treated with BamHI and EcoRI and alkaline phosphatase to yield fragment 3 (350 bp).

**Fragment 3**

```
        EcoRI                              SmaI     BamHI
   5'  |___|_____|_____|__  3'
          |_____|
          DDDDDDEEEEEFFFFFFFFFFFFFFFFFFFFFFFFFFFFFFF
```

(b) pKC7 (5.8 kb) is treated with BamHI and EcoRI to yield fragment 4 (4.0 kb).

**Fragment 4**

```
        BamHI        SalI                        EcoRI
   5'  |___|_____|_____|
                             |
                           AmpR
```

(c) Fragments 3 and 4 are ligated to form pSK3 (4.3 kb).

**pSK3**

```
                            EcoRI              SmaI   BamHI    SalI
       *5'  _____|_____|_____|_____|___| 3'*
                 |                DDDDDDEEEEEFFFFFFFFFFF
               AmpR
```

D = Trp promoter/operator.
E = TrpL ribosome binding site.
F = trpLE and restriction sites.
AmpR = Ampicillin resistance.

CHART 3. CONSTRUCTION OF pSK4.

(a) pSK3 is cut with EcoRI and BamHI to isolate fragment 5 (322 bp).

**Fragment 5**

```
        EcoRI      TaqI'      TaqI''          TaqI'''        SmaI  BamHI
   5'  |_____|_____|____AAAAAAGGGTAT|CGACAATG_____|_____| 3'
                     DDDDDDDDDDDDDDDDDDDEEE
            ←            278 bp              →
```

(b) A partial TaqI digestion yields the 278 bp fragment 6 EcoRI to TaqI''' and other fragments.
(c) Fragment 6 is cloned into pBR322, cut with EcoRI and ClaI to yield pSK4 (4.6 kb).

**pSK4**

```
            EcoRI                   ClaI     BamHI      SalI
    *5'  ___|_____|_____|_____|_____ 3'*
           |      DDDDDDDDDDDDDDEEEE
         AmpR
```

D = Trp promoter/operator.
E = Shine-Dalgarno region.

CHART 4. CONSTRUCTION OF pTPA 3' cDNA

(a) Plasmid pTPAH (5.7 kb) is obtained by cutting pBR322 with PstI, tailing and inserting the 3' region of TPAcDNA having positions 1250 to 2530.

```
                PstI    SacI  EcoRI     PstI    PvuI
      *  _____|_____|_____|_____|_____|_____  *
             |        PPPPP AAAAA AAAANNNNN
           TetR
```

(b) Plasmid pTPA80-1 (5.4 kb) is obtained by cutting pBR322 with PstI, tailing and inserting cDNA of TPA having positions 550-1600.

```
              PstI              SacI  PstI      PvuI
  *            |                 |     |         |          *
  |_____
           |        PPPPPPPPPPPPP AAAAAA
           |
         TetR
```

(c) Plasmid pTPAH and pTPA80-1 are cut with SacI and PvuI and fragments 7 (1251 bp) and 8 (5.1 kb) respectively are gel isolated. The fragments are treated with bacterial alkaline phosphatase and ligated with T4 to form pTPA3' cDNA (6.3 kb) having bases 550-2530 of the TPAcDNA.

```
         PvuII  PstI   HgaI    EcoRI    EcoRI AatII   PstI   PvuI
  *        |     |       |       |        |     |       |     |      *
  |_____
        |      PPPPPPPPPPPPPPPPPPAAAAAAAAAAAAAANNNNNNNNNN      |
        |           |_____|
      TetR          |       |       |        |              |
                   550     590     802     1274            2530
```

TetR = Tetracycline resistance.
P = 550-802 base positions of TPAcDNA.
A = 803-2530 base positions of TPAcDNA.
N = Untranslated 3' region of TPAcDNA.

CHART 5. CONSTRUCTION OF pTPAcDNA.

(a) Plasmid pTPA 5' cDNA is obtained by cutting pBR322 with PstI, tailing and inserting the 5' region of TPAcDNA positions 1-750.

```
         TaqI  PstI  HgaI BglII NarI    HgaI  PstI    TaqI
  *        |    |     |    |     |        |     |       |        *
  |_____
              |     TTTTTTTTTTTTTTTTTTTTTTTTTTTTTTT
```

(b) Plasmid pTPA 5' is cut with TaqI to yield fragment 9 (2194 bp) with is gel isolated and cut with BglII and HgaI to yield fragment 10 (405 bp) containing bases 188 to 593 of TPAcDNA (the mature TPA protein).

**Fragment 10**

```
         BglII                              HgaI
          |                                  |
          |_____|
              TTTTTTTTTTTTTTTTTTTTTTTTTTTTTT
```

(c) Plasmid pTPA 3' cDNA (Chart 4) is cut with PvuII and EcoRI and fragment 11 (1748 bp) is gel isolated. Fragment 11 is cut with HgaI to yield fragment 12 (209 bp) which is gel isolated and contains bases 594 to 802.

**Fragment 11**

```
        PvuI    PstI     HgaI    EcoRI
        |_____|_____|_____|
                 PPPPPPPPPPPP
                 ←   209   →
```

(d) Plasmid pTPA 3' cDNA is cut with PvuI, the linear 6.3 kb fragment is treated with T4 polymerase to blunt the ends, and partially digested with EcoRI to yield fragment 13 (1871 kb) containing bases 802 to 2530 plus 123 bp of pBR322.

**Fragment 13**

```
EcoRI   ScaI    EcoRI   BglII   AatII    PstI    PvuI
|_____|_____|_____|_____|_____|_____|
  AAAAAAAAAAAAAA NNNNNNNNNNNNNNNNNNNNNNNNN
```

(e) Plasmid pKC7 is cut with BglII and SmaI, treated with bacterial alkaline phosphatase and fragment 14 (4.8 kb) is gel isolated.

**Fragment 14**

```
     BglII              ScaI              SmaI
     |_____|_____|
                        |
                      AmpR
```

(f) Fragments 10, 12, 13 and 14 are pooled and ligated using T4 ligase to yield pTPAcDNA (7.4 kb) containing the nature TPAcDNA.

```
  ScaI HindIII BglII NarI HgaI  ScaI  EcoRI EcoRI BglII AatII
* |_____|_____|_____|____|_____|_____|_____|_____|_____|_____ *
  |               TTTTTTTTTT PPPPP AAAAAAAAAANNNNNNNNNNNNNNNNNNNNN
AmpR
```

AmpR = Ampicillin resistance.
T = 5' portion of TPA cDNA.
P = Middle portion of TPA cDNA.
A = 3' portion of TPA cDNA.
N = Untranslated 3' portion of TPA cDNA.

CHART 6. CONSTRUCTION OF pTPA-B1

(a) Plasmid pSK4 is cut with Cla1 and SphI to yield fragment 15 (4.1 kb) which is isolated and ligated to Block 1 containing the finger domain through a ClaI/XbaI linker containing ATG.

**Fragment 15**

(a)

SphI                     EcoRI         ClaI

DDDDEEEE

Block 1 (150 kb)

XbaI         SphI

FFFFFFFFFF

Linker (ClaI) CGATAAGCTATGT

TATTCGATACAGATC (XbaI)

(b) Fragments 15 and Block 1 are ligated through the linker to yield pTPA-B1 (4.25 kb).

**pTPA-B1**

*5'

EcoRI     ClaI       Xba      SphI     ScaI              3'*

DDDEEEE              FFFFFF

ATG                       AmpR

D = Trp promoter/operator.
E - = TrpL ribosome binding site.
F = Finger domain.
AmpR = Ampicillin resistance.
ATG = Initiation codon.

CHART 7. CONSTRUCTION OF pTPA-B1,2

(a) Plasmid pTPA-B1 is cut with EcoRI and SphI to yield fragment 16 (450 bp) and Block 2 (100 bp) containing the growth factor domain, and is isolated from the appropriate clone using EcoRI and SphI.

**Fragment 16**

EcoRI             ClaI    Xba       SphI

FFFFFFFF

P/O    RbS     ATG

**Block 2**

SphI      ClaI

GGGGGGGGGG

37

(b) Fragment 16 and Block 2 are ligated to fragment 17 (4.35 kb) derived from an EcoRI/ClaI digestion of pBR322 to yield pTPA-B1,2 (4.8 kb).

### Fragment 17

### pTPA-B1,2

P/O  =  Trp promoter/operator.
RbS  =  Trp ribosome binding site.
ATG  =  Initiation codon.
F  =  Finger domain.
G  =  Growth factor domain.
AmpR  =  Ampicillin resistance.
Ori  =  Replication origin of PBR322.

CHART 8. CONSTRUCTION OF pTPA-B1,2(a)

(a) Plasmid pTPA-B 1,2 is cut with XbaI and EcoRI to yield fragment 18 (4.5 kb) which was isolated and ligated to a linker containing a HindIII site and BglII site to yield pTPA-B1,2(a) (4.5 kb).

**Fragment 18**

```
        Xba      SphI    ClaI              ScaI      EcoRI
         |        |       |                 |         |
         |_____|_____|_____|_____|
             FFFFF   GGGGG                   |
                                             |
                                            AmpR
```

```
                           HindIII
                              |
        Linker (EcoRI) AATTCAAGCTTAGAT
                           GTTCGAATCTAGATC (XbaI)
                                   |
                                 BglII
```

**pTPA-B1,2(a)**

```
        BglII  Xba    SphI     ClaI    BamHI ScaI  EcoRI  HindIII
         |      |       |         |       |    |     |       |
      *__|_____|_____|_____|_____|____|_____|_____|___*
                 FFFFFF   GGGGGG                |
                                                |
                                               AmpR
```

F = Finger.
G = Growth factor region.
AmpR = Ampicillin resistance.

CHART 9. CONSTRUCTION OF pTPA-B1,2,3

Plasmid pTPA-B1,2(a) is cut with ClaI/BamHI and fragment 19 (4.0 kb) is isolated and ligated with Block 3 (270 bp) containing kringle 1 and a ClaI site upstream and a BamHI site downstream to yield pTPA3 (4.3 kb).

**Fragment 19**

```
  BamHI   ScaI      EcoRI  HindIII  BglII     Xba   SphI  ClaI
  |_____|_____|_____|_____|_____|_____|_____|____
          |                                             FFFFF GGGGG
        AmpR
```

```
          ClaI                        BamHI
          |_____|
          KKKKKKKKKKKKKKKK
```

**pTPA-B1,2,3**

```
       EcoRI HindIII BglII   Xba    SphI    ClaI    BamHI   ScaI
  *  ___|_____|_____|_____|_____|_____|_____|_____|_____  *
                              FFFFFF GGGGGG KKKKKK          |
                                                          AmpR
```

F = Finger domain.
G = Growth factor domain.
K = Kringle 1.
AmpR = Ampicillin resistance.

CHART 10. CONSTRUCTION OF pTPA-B1,2,3,4(a)

(a) Plasmid pTPAcDNA (Chart 5) is cut with ScaI and fragment 20 (6.0 kb) containing the 3' portion of the TPA gene is gel isolated.

**Fragment 20**

```
     ScaI      EcoRI     BglII    AatII        ScaI
     |_____|_____|_____|_____|
     |aaaaaaaaaaaNNNNNNNNNNNNNNNNNNNNN           |
                                              AmpR
```

(b) Plasmid pTPA-B1,2,3 (Chart 9) is cut with ScaI and BamHI and fragment 21 (1100 bp) is gel isolated.

**Fragment 21**

```
     ScaI      Xba     SphI     ClaI          BamHI
     |_____|_____|_____|_____|
     |          FFFFFFF GGGGGGG KKKKKKKKKKKK
   AmpR
```

(c) From the clone containing block 4 isolate a BamHI/ScaI fragment 4a (230 bp) containing the Kringle 2 region.

**Fragment 4a**

```
      BamHI                    ScaI
      |_____|
       |kkkkkkkkkkkkkkkkkkkkkkk|
```

(d) Fragments 20, 21 and 4a are ligated to form pTPA-B1,2,3,4a.

```
    HindIII XbaI    SphI    ClaI    BamHI   ScaI    BalI    AatII
     SacI | BglII | HpaI | EcoRV | SmaI | HpaI | EcoRI | BglII |
   *  ____|____|___|___|____|____|___|____|___|____|___|____|____  *
         |            FFF     GGG         KK     kkk aaaaaaNNNNNNNNNNNNNN
     AmpR
```

(e) Plasmid pTPA-B1,2,3,4a is cut with HindIII and AatII and the 2.2 kb prototype TPA gene is subcloned into pUC19 similarly digested with HindIII and AatII to yield pTPA-B1,2,3,4a (4.2 kb). Plasmid pTPA-B1,2,3,4a is symbolically similar to the pTPA-B1,2,3,4a but has a more convenient arrangement of restriction sites.
AmpR = Ampicillin resistance.
F = Finger domain.
G = Growth factor domain.
K = Kringle 1 domain.
k = Kringle 2 domain.
a = Active site.
N = Untranslated 3' portion of TPAcDNA.

CHART 11. CONSTRUCTION OF pTPA-B 1,2,3,4

(a) Plasmid pTPA-B1,2,3,4a is cut with EcoRI and BamHI. The large fragment (3.7 kb) is ligated to block 4 (560 bp) obtained from the appropriate clone also cut with EcoRI (partial digestion) and BamHI to yield pTPA-B1,2,3,4.

```
    HindIII  XbaI    SphI    ClaI    BamHI  ScaI    XmaII   EcoRI BglII
   ScaI | BglII | HpaI | EcoRV | SmaI | HpaI | MstI | SphI |BalI |AatII
   * _|___|____|___|____|___|____|___|____|___|____|__|__|__|____ *
      |            FFF     GGG         KK     kk            AAAAAANNNNNNNN
    AmpR
```

AmpR = Ampicillin resistance.
F = Finger domain.
G = Growth factor domain.
K = Kringle 1 domain.
k = Kringle 2 domain.
A - 3' portion of TPAcDNA.
N = Untranslated 3' portion of TPAcDNA.

CHART 12. CONSTRUCTION OF pFK2K2A

(a) Plasmid pTPA-B1,2,3,4a is cut with HpaI to yield fragment 22 (6.5 kb) which is isolated from a gel.

**Fragment 22**

```
HpaI MstI   EcoRI    BalI  BglII              BglII XbaI HpaI
 |    |       |        |     |                   |    |   |
 |----kkk----AAAAAANNNNN-----|-------------------|----|---|
                                    |                  |    FFFF
                                   Ori               AmpR
```

(b) Plasmid pTPA-B1,2,3,4 is cut with HpaI and MstI to yield fragment 23 (506 bp) containing kringle 2.

```
       HpaI        MstI
        |           |
        |---kkkkkkkkkk---|
```

(c) Fragments 22 and 23 are ligated together to yield pFK2K2A (7.1 kb).

```
              BglII XbaI  HpaI            BalI  BglII
               |    |      |               |     |
 *  -----------|----|------|---------------|-----|--  *
        |           |      FFFFFF kkk   kkk AAAAA NNNNN
       Ori        AmpR
```

Ori = Replication origin for pBR322.
AmpR = Ampicillin resistance.
k = Kringle 2 domain.
F = Finger domain.
A = Active site.
N = Noncoding 3' sequence.

CHART 13. CONSTRUCTION OF pTPAExp 1

(a) Plasmid pTPA-B1,2 (Chart 7) is cut with XbaI and BamHI and fragment 24 (4.2 kb) which is gel isolated.

**Fragment 24**

```
   BamHI            EcoRI ClaI    XbaI
    |                 |    |        |
    |-----------------|----|--------|
         |                P/O RbS ATG
        AmpR
```

(b) Plasmid pFK2K2A (Chart 12) is cut with XbaI and BglII to yield fragment 25 (2.0 kb) which is gel isolated.

**Fragment 25**

```
   XbaI  HpaI       MstI  SphI    BalI BglII
    |     |          |     |        |    |
    |-----|----------|-----|--------|----|
     FFFFF kkkk  kkkk        AAAAAANN NNNN
```

(c) Fragments 24 and 25 are ligated to form pTPAExp 1 (6.2 kb); the BamHI and BgIII are complementary but are not restored.

**pTPAExp 1**

```
              EcoRI      ClaI  XbaI  HpaI         MstI  SphI  BalI
     5'*       |          |     |     |            |     |     |        3'*
               |    |     |     |     FFFFF kkkk    kkkk   AAAAAA NNNN
             AmpR trpP/O RbS ATG
```

trpP/O - Tryptophage promoter/operator.
RbS = Ribosome binding site.
k = Kringle 2.
F = Finger domain.
A = Active site.
N = Noncoding 3' sequence.
AmpR = Ampicillin resistance.
ATG = Initiation codon.

CHART 14. CONSTRUCTION OF pyRep3'B

(a) Plasmid pYIP31 is cut with SmaI and treated with bacterial alkaline phosphatase to yield fragment 26.

**Fragment 26**

```
            SmaI     HindIII EcoRI   HindIII
     5'      |          |     |        |          3'
            |_____|_____|_____|_____
                                        uuuuuuuu
```

(b) The 2μ plasmid of yeast is cut with PstI and XbaI and treated with Klenow enzyme. The resulting fragment 27 (1.3 kb) is isolated.

**Fragment 27**

```
            PstI                XbaI
     5'      |                   |   3'
            |_____|
               RRRRRRRRRRRRRRRR
```

(c) Fragments 26 and 27 are ligated to yield pyRep3'B (6.7 kb).

```
              EcoRI       HindIII         HindIII
     *5'        |           |               |          3'*
             __|_____|_____|_____
                           uuuuuuuuRRRRRRRRRR
```

u = URA3
R = Sequence spanning the RepIII and replication origin.

CHART 15. CONSTRUCTION OF PLASMID pGG400

(a) Plasmid pBR322 is cut with EcoRI and PvuII and ends filled in with Klenow enzyme to yield fragment 28 (2.3 kb).

**Fragment 28**

```
          PvuII                              EcoRI
     5'    |_____|  3'
                                  |
                                AmpR
```

(b) Plasmid pML21 is cut with PvuII to yield fragment 29 (1.7 kb).

**Fragment 29**

```
          PvuII                              PvuII
     5'    |_____|  3'
                     |
                    KmR
```

(c) Fragments 28 and 29 are ligated using T4 to yield pGG400 (4.0 kb).

```
          EcoRI                   XhoI   PvuII
   *5'_____|_____|_____|_____  3'*
                            |            |
                           KmR          AmpR
```

AmpR = Ampicillin resistance.
KmR = Kanamycin resistance.

CHART 16. CONSTRUCTION OF pADHt

(a) Plasmid pGG400 is cut with XhoI and PvuII, treated with Klenow enzyme, and the resulting fragment 30 (3.5 kb) is isolated.

**Fragment 30**

```
     PvuII                EcoRI              XhoI
     |_____|_____|
              |                        |
            AmpR                      KmR
```

(b) Plasmid pADHBC is cut with HincII and BamHI, the ends are filled with T4 ligase and the resulting fragment 31 (0.36 kb) is isolated.

**Fragment 31**

```
     HincII(XhoI)  SphI              BamHI
     |_____|_____|
           HHHHHHHHHHHHHHHHHHHHHHHHHHHHHH
```

(c) Fragments 30 and 31 are ligated using T4 ligase to obtain pADHt (3.86 kb).

```
    EcoRI HindIII SmaI    XhoI      SphI    BamHI
*    |    |     |         |         |        |         *
     ————————————————————————————————————————————————
                                HHHHHHHHHHHHHHHH|
                                                AmpR
```

AmpR = Ampicillin resistance.
KmR = Kanamycin resistance.
H = ADHt 3' end.

CHART 17. CONSTRUCTION OF PLASMID pADHt-RepIII

(a) Plasmid pADHt is modified by restriction with BamHI and ligation of an 8-mer SalI linker into the filled BamHI site.

```
       EcoRI       HindIII         XhoI  SphI   SalI
*       |           |               |    |       |        *
        ———————————————————————————————————————————————
                                    HHHHHHHHHHHHHH|
                                                  AmpR
```

(b) Plasmid pADHt (modified) is cut with SphI and the ends filled in with T4 ligase to yield fragment 32 (3.8 kb).
(c) Plasmid pyRep31B (Chart 16) is cut with HindIII and the ends filled in with T4 ligase to yield fragment 33 (2.4 kb).

```
    HindIII                          HindIII
    |                                |
    ——————————————————————————————————
       uuuuuuuuuuuuuuuuuuuRRRRRRRRRRRRRRRR
```

(d) Fragments 32 and 33 are ligated using T4 ligase to obtain pADHt-RepIII (6.2 kb).

```
      EcoRI SmaI  HindIII XhoI                    SalI
*      |    |     |       |                        |       *
       —————————————————————————————————————————————————
                          HHHHHH uuuuRRRRHHHH|
                                             AmpR
```

AmpR = Ampicillin resistance.
H = ADHt 3'end.
u = Ura3
R = RepIII and origin of replication

CHART 18. CONSTRUCTION OF pα1-ADHt

(a) Plasmid pADHt-RepIII (Chart 15) is cut with EcoRI and HindIII and fragment 34 (5.3 kb) is isolated.

45

**Fragment 34**

```
     HindIII        XhoI              SalI              EcoRI
        |             |                 |                 |
        |_____|_____|_____|
                         HHHuuuuRRRRHHHH        |
                                              AmpR
```

(b) Plasmid pα1 is restricted with EcoRI and HindIII to yield the MFα1 gene containing fragment 35 having 1.2 kb.

**Fragment 35**

```
              EcoRI                         HindIII
        5'      |                              |      3'
                |_____|
                         |
                       MFα1
```

(c) Fragments 34 and 35 are ligated using T4 ligase to obtain pα1-ADHt (6.5 kb).

```
          EcoRI        HindIII           XhoI              SalI
    *5'     |             |                |                 |          3'*
    _____|_____|_____|_____|_____
            |                                 HHHuuuuRRRHHHHH    |
          MFα1                                                  AmpR
```

AmpR = Ampicillin resistance.
H = ADHt 3' end.
u = Ura3.
R = RepIII and origin of replication
MFα1 = Promoter and signal sequence for MFα1 gene.

CHART 19. CONSTRUCTION OF pα1-FK2K2A

(a) Plasmid pFK2K2A (Chart 12) is cut with BglII to obtain fragment 36 (2.0 kb) which is gel isolated.

**Fragment 36**

```
     BglII  XbaI   HpaI              MstI    BalI    BglII
       |     |      |                 |       |       |
       |_____|_____|_____|_____|_____|
          FFFFFF  kkkkkkk  kkkkkkk AAAAAAA NNNNNNN
```

(b) Plasmid pα1-ADHt is cut with HindIII and XhoI; the ends are partially filled with Klenow enzyme in the presence of bases adenosine and guanosine, and treated with Mung Bean nuclease to yield fragment 37 (5.6 kb) which is gel isolated.

46

**Fragment 37**

(c) Fragments 36 and 37 are ligated to form pα1-FK2K2A (7.6 kb).

MFα1 = Promoter and signal sequence for MFα1 gene.
F = Finger.
k = Kringle region 2.
A = Active site.
N = Untranslated 3' sequence.
H = ADHt 3' end.
u = URA3
R = RepIII and origin of replication.

CHART 20. CONSTRUCTION OF pSVCOW7

(a) Plasmid pSV2dhfr is cut with BamHI and EcoRI to obtain fragment 38 (5.0 kb).

**Fragment 38**

(b) Plasmid pλGH2R2 is cut with BamHI and EcoRI to obtain fragment 39 (2.1 kb).

**Fragment 39**

(c) Fragments 38 and 39 are ligated to yield pSVCOW7 (7.1 kb).

**pSVCOW7**

```
              EcoRI  PvuII   PstI   BamHI       PvuII   HindIII
         *      |      |       |      |           |        |        *
                ‾‾‾‾‾‾‾‾‾‾‾‾‾‾‾‾‾‾‾‾‾‾‾‾‾‾‾‾‾‾‾‾‾‾‾‾‾‾‾‾‾‾‾‾‾‾‾‾‾‾
                AAAGGGIIGGGGGGGGGG
                                        |           |        |
                                      dhfr        SV40     AmpR
```

A = Bovine growth hormone poly A tail.
G = Genomic bovine growth hormone.
I = Intron.
dhfr = Dihydrofolate reductase.
SV40 = SV40 promoter and origin of replication.
AmpR = Ampicillin resistance.

CHART 21. CONSTRUCTION OF pTPAcDNA, BamHI

(a) Plasmid pTPA5'cDNA (Chart 5, part a) is cut with Hga and fragment 40 (517 bp) is made flush with Klenow and ligated to a 10-mer BamHI linker. Fragment 40 is cut with NarI to yield fragment 41 (440 bp) having bases 78 to 518 of the TPAcDNA.

**Fragment 40**

```
         BamHI     BglII                  NarI          BamHI
           |         |                      |              |
           ‾‾‾‾‾‾‾‾‾‾‾‾‾‾‾‾‾‾‾‾‾‾‾‾‾‾‾‾‾‾‾‾‾‾‾‾‾‾‾‾‾‾‾‾‾‾‾‾
           LLLTTTTTTTTTTTTTTTTTTTTTTT
              ←            440           →
```

(b) Plasmid pTPAcDNA (Chart 5) is cut with NarI and BglII and fragment 42 (1644 bp) containing the 3' portion of TPAcDNA which is gel isolated.

**Fragment 42**

```
         NarI   HgaI       EcoRI   EcoRI          BglII
           |      |           |       |             |
           ‾‾‾‾‾‾‾‾‾‾‾‾‾‾‾‾‾‾‾‾‾‾‾‾‾‾‾‾‾‾‾‾‾‾‾‾‾‾‾‾‾‾
           PPPPPPPPPPPPPP AAAAAAAAAANNNNNNNNNN
```

(c) Plasmid pKC7 is cut with BamHI and BglII to yield fragment 43 (4.3 kb) which is gel isolated and ligated with fragments 41 and 42 to yield pTPAcDNA, BamHI (6.5 kb).

**pTPAcDNA, BamHI**

```
              BamHI  BglII              BalI   BglII
         *      |      |                  |      |              *
         ‾‾‾‾‾‾‾‾‾‾‾‾‾‾‾‾‾‾‾‾‾‾‾‾‾‾‾‾‾‾‾‾‾‾‾‾‾‾‾‾‾‾‾‾‾‾‾‾‾‾‾‾‾‾
                LLLLTTTTPPPPAAAANNNN NNNNN
                                              |
                                            AmpR
```

L = Leader sequence.
T = 5' portion of TPAcDNA.

48

P = Middle portion of TPAcDNA.
A = 3' portion of TPAcDNA.
N = Untranslated 3' portion of TPAcDNA.

CHART 22. CONSTRUCTION OF pTPA-IE-PA

(a) Plasmid pSVCOW7 is cut with EcoRI and PvuII to yield fragment 44 (600 bp) containing the polyadenylation sequence of bovine growth hormone which is gel isolated.

**Fragment 44**

EcoRI                    PvuII

(b) Plasmid pSVCOW7 (Chart 20) is cut with EcoRI and BamHI to yield fragment 45 (5.8 kb).

**Fragment 45**

BamHI                                    EcoRI

dhfr      SV40       Ori        AmpR

(c) Plasmid pTPAcDNA, BamHI (Chart 21) is cut with BamHI and BalI to yield fragment 46 (2.0 kb).

**Fragment 46**

BamHI    BglII                          BalI

LLLLLTTTTTPPPPPAAAAANNNNNNNNNNNNNNNNNNN

(d) Fragments 44, 45 and 46 are ligated to form pTPA-PA (8.4 kb).

**pTPA-PA**

       EcoRI                    BamHI  BglII      BalI
*                                                       *

LLLTTTTPPPAAANNNNNaaa

AmpR    Ori     SV40    dhfr

Plasmid pTPA-PA is cut with BamHI and the CMV-IE promoter (760 bp) from a PstI and Sau3AI digestion of the CMV genome is inserted to form pTPA-IE-PA.

**pTPA-IE-PA**

                                      BalI
                          Sau3A   Sau3A BamHI BglII
       EcoRI
*5'                                                              3'*

LLLL  TTPPAA  NNNNaaa

AmpR    Ori    SV40    dhfr    CMV

AmpR = Ampicillin resistance.
Ori = pBR322 origin of replication.
SV40/ori = Simian virus origin of replication.
Mo/dhfr = Mouse dihydrofolate reductase marker.
CMV/Prom = Cytomegalovirus promoter.
L = TPA leader sequence.
T = 5' region of TPAcDNA.
P = Middle region of TPAcDNA.
A = 3' region of TPAcDNA.
N = Untranslated 3' region of TPAcDNA.
a = Polyadenylation signal sequence.

CHART 23. CONSTRUCTION OF pTPA-IE-FK2K2A

(a) pTPA-IE-PA (Chart 22) is cut with BamHI and BglII to yield fragment 47 (120 bp) which is gel isolated.

**Fragment 47**

**BalI**        **BglII**

LLLLLLLLLLLLLLLLLLLLLLLLLLLLLLLL

(b) Plasmid pSVCOW7 is cut with EcoRI and PvuII to yield fragment 48 (600 bp) containing the polyadenylation sequence of bovine growth hormone which is gel isolated.

**Fragment 48**

**EcoRI**     **PvuII**

aaaaaaaaaaaaaaaaaa

(c) Plasmid pFK2K2A (Chart 12) is cut with BglII and BalI to obtain fragment 49 (1.7 kb) which is gel isolated.

**Fragment 49**

**BglII**   **XbaI**   **HpaI**    **MstI**     **BalI**

FFFFFF kkk    kkk AAAAAANNNNNNNN

(d) Plasmid pSVCOW7 (Chart 20) is cut with EcoRI and BamHI to yield fragment 50.

**Fragment 50**

**BamHI**        **EcoRI**

dhfr    SV40    Ori    AmpR

(e) Fragments 47, 48, 49 and 50 are ligated using T4 ligase to yield pTPAFK2K2A (8.3 kb).

50

**pTPAFK2K2A**

(f) Plasmid pTPAFK2K2A is cut with BamHI and the CMV-IE promoter (760 bp) from a PstI and Sau3AI digestion of the CMV genome is inserted to form pTPA-IE-FK2K2A.

CHART 24. CONSTRUCTION OF pAcTPA

(a) Plasmid pAc373 (7.1 kb) is cut with BamHI and treated with mung bean nuclease to create blunt ends to which BglII linkers are added. The ends are rejoined to yield pAc373, BglII.

**pAc373**

**pAc373, BglII**

(b) Plasmid pTPAcDNA, BamHI (Chart 21) is cut with BamHI and partially digested with BglII to yield fragment 51 (1.95 kb) containing an intact TPAcDNA.

**Fragment 51**

(c) Fragment 51 is inserted and ligated to the BglII site of pAc373, BglII to yield pAcTPA (9.05 kb).

**pAcTPA**

L = Leader sequence.
T = 5' portion of TPAcDNA.

P = Middle portion of TPAcDNA.
A = 3' portion of TPAcDNA.
N = Untranslated 3' portion of TPAcDNA.
AmpR = Ampicillin resistance.
Poly = Polyhedrin protein gene.

CHART 25. CONSTRUCTION OF pAc FK2K2A

(a) pAcTPA (Chart 24) is cut with BglII to yield fragment 52 (7.15 kb).

**Fragment 52**

```
      BglII      HindIII    EcoRI        EcoRV BamHI  BglII
      |          |          |            |     |      |
   ___|_____|_____|_____|_____|_____|___
                 |                              LLLLLL
               AmpR
```

(b) Plasmid pFK2K2A (Chart 12) is cut with BglII and the analog cDNA is gel isolated as fragment 53 (2.0 kb).

**Fragment 53**

```
      BglII  XbaI    HpaI              MstI    BalI    BglII
      |      |       |                 |       |       |
   ___|_____|_____|_____|_____|_____|___
          FFFFFF kkkkkkk kkkkkkk AAAAAAA NNNNNNN
```

(c) Fragments 52 and 53 are ligated to form pAcFK2K2A (9.15 kb).

```
         HindIII BamHI   BglII XbaI  HpaI   MstI   BalI   BglII
    *    |       |       |     |     |      |      |      |     *
    _____|_____|_____|_____|_____|_____|_____|_____|_____
                 |        LLLLLL      FFFFF kk  kk AAAAA NNNNN
               AmpR
```

L = Leader sequence.
F = Finger domain.
k = Kringle 2 domain.
A = Active site domain.
N = Untranslated 3' portion of TPAcDNA.
AmpR = Ampicillin resistance.

**Claims**
**Claims for the following Contracting States : BE, CH, DE, FR, GB, IT, LU, NL, SE**

1.  A TPA-like protein having a molecular weight of less than 90,000 daltons and which, by comparison with TPA whose native order is FGK1K2A, has the order FK2A, FK2K2A or FGK2A, wherein F is the finger domain, G is the growth factor domain, K1 and K2 are the kringle 1 and 2 domains, and A is an active site.

2.  A protein according to claim 1, in which the order is FK2A.

3.  DNA coding for a protein according to claim 1 or claim 2, and which contains within the nucleotide sequences encoding the interdomain regions non-native endonuclease restriction sites that are not present in the nucleotide sequences encoding the domain regions.

4. DNA according to claim 3, which comprises an XbaI site between nucleotide bases 187 and 198 of the native arrangement.

5. DNA according to claim 3 or claim 4, which comprises a HapI or SphI site or combination thereof between nucleotide bases 328 and 342 of the native arrangement.

6. DNA according to any of claims 3 to 5, which comprises an EcoRV, ClaI or SmaI site or combination thereof between nucleotide bases 442 and 462 of the native arrangement.

7. DNA according to any of claims 3 to 6, which comprises a BamHI or HpaI site or combination thereof between nucleotide bases 709 and 726 of the native arrangement.

8. DNA according to any of claims 3 to 7, which comprises a MstHI, SphI or XmaIII site or combination thereof between nucleotide bases 973 and 997 of the native arrangement.

9. DNA according to any of claims 3 to 8, in which the sequences of nucleotides both upstream and downstream from the sequence encoding the TPA-like protein render the DNA capable of being maintained by cells of a suitable host.

10. DNA according to claim 9, capable of permitting the cells to express the TPA-like protein.

11. DNA according to claim 9 or claim 11, in which the host is yeast, eukaryotic or Escherichia sp.

12. DNA according to claim 11, in which the cells are Chinese hamster ovary cells.

13. DNA according to claim 10, in which the host is an insect.

14. DNA according to claim 13, in which the insect is Bombyx mori or Spodoptera frugiperda.

15. Cells containing DNA according to claim 10, in which the protein can be expressed, which are cells of E. coli, yeast, an insect or a mammal.

**Claims for the following Contracting States : AT, ES, GR**

1. A process for preparing a TPA-like protein having a molecular weight of less than 90,000 daltons and which, by comparison with TPA whose native order is FGK1K2A, has the order FK2A, FK2K2A or FGK2A, wherein F is the finger domain, G is the growth factor domain, K1 and K2 are the kringle 1 and 2 domains, and A is an active site,
   which comprises expressing the TPA-like protein from cells of a suitable host containing DNA that renders the host capable of maintaining the DNA and expressing the protein.

2. A process according to claim 1, in which the order is FK2A.

3. A process according to claim 1 or claim 2, in which the DNA contains within the nucleotide sequences encoding the interdomain regions non-native endonuclease restriction sites that are not present in the nucleotide sequences encoding the domain regions.

4. A process according to claim 3, in which the DNA comprises an XbaI site between nucleotide bases 187 and 198 of the native arrangement.

5. A process according to claim 3 or claim 4, in which the DNA comprises a HapI or SphI site or combination thereof between nucleotide bases 328 and 342 of the native arrangement.

6. A process according to any of claims 3 to 5, in which the DNA comprises an EcoRV, ClaI or SmaI site or combination thereof between nucleotide bases 442 and 462 of the native arrangement.

**7.** A process according to any of claims 3 to 6, in which the DNA comprises a BamHI or HpaI site or combination thereof between nucleotide bases 709 and 726 of the native arrangement.

**8.** A process according to any of claims 3 to 7, in which the DNA comprises a MstHI, SphI or XmaIII site or combination thereof between nucleotide bases 973 and 997 of the native arrangement.

**9.** A process according to any preceding claim, in which the host is yeast, eukaryotic or Escherichia sp.

**10.** A process according to claim 11, in which the cells are Chinese hamster ovary cells.

**11.** A process according to any of claims 1 to 8, in which the host is an insect.

**12.** A process according to claim 11, in which the insect is Bombyx mori or Spodoptera frugiperda.

**Patentansprüche**
**Patentansprüche für folgende Vertragsstaaten : BE, CH, DE, FR, GB, IT, LU, NL, SE**

**1.** TPA-ähnliches Protein mit einem Molekulargewicht von weniger als 90000 Dalton und, das im Vergleich mit TPA, dessen nativer Aufbau FGK1K2A ist, den Aufbau FK2A, FK2K2A oder FGK2A hat, worin F die Finger-Domäne ist, G die Wachstumsfaktor-Domäne ist, K1 und K2 die Kringle 1- und 2-Domänen sind und A eine aktive Stelle ist.

**2.** Protein nach Anpruch 1 mit dem Aufbau FK2A.

**3.** DNA, codierend für ein Protein nach Anspruch 1 oder 2 und enthaltend in den die Zwischendomänen-Bereiche codierenden Nukleotidsequenzen nicht-native Endonuklease-Restriktionsstellen, die in den die Domänen-Bereiche codierenden Nukleotidsequenzen nicht vorliegen.

**4.** DNA nach Anspruch 3, enthaltend eine XbaI-Stelle zwischen den Nukleotidbasen 187 und 198 der nativen Anordnung.

**5.** DNA nach Anspruch 3 oder 4, enthaltend eine HpaI- oder SphI-Stelle oder eine Kombination davon zwischen den Nukleotidbasen 328 und 342 der nativen Anordnung.

**6.** DNA nach einem der Ansprüche 3 bis 5, enthaltend eine EcoRV-, ClaI- oder SmaI-Stelle oder eine Kombination davon zwischen den Nukleotidbasen 442 und 462 der nativen Anordnung.

**7.** DNA nach einem der Ansprüche 3 bis 6, enthaltend eine BamHI- oder HpaI-Stelle oder eine Kombination davon zwischen den Nukleotidbasen 709 und 726 der nativen Anordnung.

**8.** DNA nach einem der Ansprüche 3 bis 7, enthaltend eine MstHI-, SphI- oder XmaIII-Stelle oder eine Kombination davon zwischen den Nukleotidbasen 973 und 997 der nativen Anordnung.

**9.** DNA nach einem der Ansprüche 3 bis 8, worin die Nukleotidsequenzen upstream and downstream der das TPA-ähnliche Protein codierenden Sequenz die DNA befähigen, durch Zellen eines geeigneten Wirts aufrechterhalten zu werden.

**10.** DNA nach Anspruch 9 mit der Fähigkeit, die Zellen das TPA-ähnliche Protein exprimieren zu lassen

**11.** DNA nach Anspruch 9 oder 10, worin der Wirt Hefe, eine eukaryotische oder Escherichia Spezies ist.

**12.** DNA nach Anspruch 11, worin die Zellen Eierstockzellen von Chinesischem Hamster sind.

**13.** DNA nach Anspruch 10, worin der Wirt ein Insekt ist.

**14.** DNA nach Anspruch 13, worin das Insekt Bombyx mori oder Spodoptera frugiperda ist.

**15.** Zellen, enthaltend DNA nach Anspruch 10, in denen das Protein exprimiert werden kann, nämlich

EP 0 231 624 B1

Zellen von E.coli, Hefe, einem Insekt oder einem Säugetier.

**Patentansprüche für folgende Vertragsstaaten : AT, ES, GR**

1.  Verfahren zur Herstellung eines TPA-ähnlichen Proteins mit einem Molekulargewicht von weniger als 90000 Dalton und, das im Vergleich mit TPA, dessen nativer Aufbau FGK1K2A ist, den Aufbau FK2A, FK2K2A oder FGK2A hat, worin F die Finger-Domäne ist, G die Wachtstumsfaktor-Domäne ist, K1 und K2 die Kringle 1- und 2-Domänen sind und A eine aktive Stelle ist, bei dem das TPA-ähnliche Protein von Zellen eines geeigneten, eine DNA enthaltenden Wirts exprimiert wird, wobei die DNA den Wirt zur Aufrechterhaltung der DNA und zur Expression des Proteins befähigt.

2.  Verfahren nach Anspruch 1, worin das Protein den Aufbau FK2A hat.

3.  Verfahren nach Anspruch 1 oder 2, worin die DNA in den die Zwischendomänen-Bereiche codierenden Nukleotidsequenzen nicht-native Endonuklease- Restriktionsstellen aufweist, die in den die Domänen-Bereiche codierenden Nukleotidsequenzen nicht vorliegen.

4.  Verfahren nach Anspruch 3, worin die DNA eine XbaI-Stelle zwischen den Nukleotidbasen 187 und 198 der nativen Anordnung umfaßt.

5.  Verfahren nach Anspruch 3 oder 4, worin die DNA eine HpaI- oder SphI-Stelle oder eine Kombination davon zwischen den Nukleotidbasen 328 und 342 der nativen Anordnung umfaßt.

6.  Verfahren nach einem der Ansprüche 3 bis 5, worin die DNA eine EcoRV-, ClaI- oder SmaI-Stelle oder eine Kombination davon zwischen den Nukleotidbasen 442 und 462 der nativen Anordnung umfaßt.

7.  Verfahren nach einem der Ansprüche 3 bis 6, worin die DNA eine BamHI- oder HpaI-Stelle oder eine Kombination davon zwischen den Nukleotidbasen 709 und 726 der nativen Anordnung umfaßt.

8.  Verfahren nach einem der Ansprüche 3 bis 7, worin die DNA eine MstHI-, SphI- oder XmaIII-Stelle oder eine Kombination davon zwischen den Nukleotidbasen 973 und 997 der nativen Anordnung umfaßt.

9.  Verfahren nach einem der vorhergehenden Ansprüche, worin der Wirt Hefe, eine eukaryotische oder Escherichia Spezies ist.

10. Verfahren nach Anspruch 11, worin die Zellen Eierstockzellen von Chinesischem Hamster sind.

11. Verfahren nach einem der Ansprüche 1 bis 8, worin der Wirt ein Insekt ist.

12. Verfahren nach Anspruch 11, worin das Insekt Bombyx mori oder Spodoptera frugiperda ist.

**Revendications**
**Revendications pour les Etats contractants suivants : BE, CH, DE, FR, GB, IT, LU, NL, SE**

1.  Protéine analogue au TPA, ayant un poids moléculaire inférieur à 90 000 daltons et qui, par comparaison au TPA dont l'ordre naturel est FGK1K2A, possède l'ordre FK2A, FK2K2A ou FGK2A, F étant le domaine en forme de doigt, G étant le domaine du facteur de croissance, K1 et K2 étant les domaines des kringles 1 et 2 et A représentant un site actif.

2.  Protéine suivant la revendication 1, dans laquelle l'ordre est FK2A.

3.  ADN codant pour une protéine suivant la revendication 1 ou la revendication 2, et qui contient dans les séquences de nucléotides codant pour les régions d'interdomaines des sites de restriction par endonucléases non naturels qui ne sont pas présents dans les séquences de nucléotides codant pour les régions des domaines.

4.  ADN suivant la revendication 3, qui comprend un site XbaI entre les bases de nucléotides 187 et 198 de la configuration naturelle.

55

**5.** ADN suivant la revendication 3 ou la revendication 4, qui comprend un site HapI ou SphI ou bien leur association entre les bases de nucléotides 328 et 342 de la configuration naturelle.

**6.** ADN suivant l'une quelconque des revendications 3 à 5, qui comprend un site EcoRV, ClaI ou SmaI ou bien leur association entre les bases de nucléotides 442 et 462 de la configuration naturelle.

**7.** ADN suivant l'une quelconque des revendications 3 à 6, qui comprend un site BamHI ou HpaI ou bien leur association entre les bases de nucléotides 709 et 726 de la configuration naturelle.

**8.** ADN suivant l'une quelconque des revendications 3 à 7, qui comprend un site MstHI, SphI ou XmaIII ou bien leur association entre les bases de nucléotides 973 et 997 de la configuration naturelle.

**9.** ADN suivant l'une quelconque des revendications 3 à 8, dans lequel les séquences de nucléotides en amont et en aval de la séquence codant pour la protéine analogue au TPA rendent l'ADN capable d'être conservé par les cellules d'un hôte convenable.

**10.** ADN suivant la revendication 9, capable de permettre l'expression de la protéine analogue au TPA par les cellules.

**11.** ADN suivant la revendication 9 ou la revendication 10, dans lequel l'hôte est une levure, un organisme eucaryotique ou Escherichia sp.

**12.** ADN suivant la revendication 11, dans lequel les cellules sont des cellules d'ovaires de hamster chinois.

**13.** ADN suivant la revendication 10, dans lequel l'hôte est un insecte.

**14.** ADN suivant la revendication 13, dans lequel l'insecte est Bombyx mori ou Spodoptera frugiperda.

**15.** Cellules contenant un ADN suivant la revendication 10, dans lesquelles la protéine peut être exprimée, qui sont des cellules de E. coli, d'une levure, d'un insecte ou d'un mammifère.

**Revendications pour les Etats contractants suivants : AT, ES, GR**

**1.** Procédé de préparation d'une protéine analogue au TPA, ayant un poids moléculaire inférieur à 90 000 daltons et qui, par comparaison au TPA dont l'ordre naturel est FGK1K2A, possède l'ordre FK2A, FK2K2A ou FGK2A, F étant le domaine en forme de doigt, G étant le domaine du facteur de croissance, K1 et K2 étant les domaines des kringles 1 et 2 et A étant un site actif,
qui consiste à provoquer l'expression de la protéine analogue au TPA par des cellules d'un hôte convenable, ces cellules contenant de l'ADN qui rend l'hôte capable de conserver l'ADN et d'exprimer la protéine.

**2.** Procédé suivant la revendication 1, dans lequel l'ordre est FK2A.

**3.** Procédé suivant la revendication 1 ou la revendication 2, dans lequel l'ADN contient dans les séquences de nucléotides codant pour les régions d'interdomaines des sites de restriction par endonucléases non naturels qui ne sont pas présents dans les séquences de nucléotides codant pour les régions des domaines.

**4.** Procédé suivant la revendication 3, dans lequel l'ADN comprend un site XbaI entre les bases de nucléotides 187 et 198 de la configuration naturelle.

**5.** Procédé suivant la revendication 3 ou la revendication 4, dans lequel l'ADN comprend un site HapI ou SphI ou bien leur association entre les bases de nucléotides 328 et 342 de la configuration naturelle.

**6.** Procédé suivant l'une quelconque des revendications 3 à 5, dans lequel l'ADN comprend un site EcoRV, ClaI ou SmaI ou bien leur association entre les bases de nucléotides 442 et 462 de la configuration naturelle.

56

**7.** Procédé suivant l'une quelconque des revendications 3 à 6, dans lequel l'ADN comprend un site BamHI ou HpaI ou bien leur association entre les bases de nucléotides 709 et 726 de la configuration naturelle.

**8.** Procédé suivant l'une quelconque des revendications 3 à 7, dans lequel l'ADN comprend un site MstHI, SphI ou XmaIII ou bien leur association entre les bases de nucléotides 973 et 997 de la configuration naturelle.

**9.** Procédé suivant l'une quelconque des revendications précédentes, dans lequel l'hôte est une levure, un organisme eucaryotique ou Escherichia sp.

**10.** Procédé suivant la revendication 9, dans lequel les cellules sont des cellules d'ovaires de hamster chinois.

**11.** Procédé suivant l'une quelconque des revendications 1 à 8, dans lequel l'hôte est un insecte.

**12.** Procédé suivant la revendication 11, dans lequel l'insecte est Bombyx mori ou Spodoptera frugiperda.